(19) Europäisches Patentamt
European Patent Office
Office européen des brevets

(11) Numéro de publication : **0 508 842 B1**

(12) # FASCICULE DE BREVET EUROPEEN

(45) Date de publication du fascicule du brevet : **10.08.94 Bulletin 94/32**

(51) Int. Cl.$^5$ : **C07C 233/25,** C07C 233/26, C07C 233/27, A61K 31/16

(21) Numéro de dépôt : **92400576.2**

(22) Date de dépôt : **06.03.92**

(54) **Nouveaux dérivés d'acylaminophénol, leur procédé de préparation et les compositions pharmaceutiques qui les contiennent.**

(30) Priorité : **08.03.91 FR 9102800**

(43) Date de publication de la demande : **14.10.92 Bulletin 92/42**

(45) Mention de la délivrance du brevet : **10.08.94 Bulletin 94/32**

(84) Etats contractants désignés : **AT BE CH DE DK ES FR GB GR IT LI LU NL PT SE**

(56) Documents cités :
**EP-A- 0 242 610**
**US-A- 4 716 175**

(73) Titulaire : **ADIR ET COMPAGNIE**
**1 rue Carle Hébert**
**F-92415 Courbevoie Cédex (FR)**

(72) Inventeur : **Malen, Charles**
**3 allée Traversière**
**F-94260 Fresnes (FR)**
Inventeur : **Lacoste, Jean-Michel**
**103, rue Brancas**
**F-92310 Sevres (FR)**
Inventeur : **Vilaine, Jean-Paul**
**21, rue des Vallées**
**F-92290 Chatenay-Malabry (FR)**
Inventeur : **Lenaers, Albert**
**20 allée des Martinets**
**F-78510 Triel sur Seine (FR)**

**EP 0 508 842 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés d'acylaminophénol, leur procédé de préparation et les compositions pharmaceutiques qui les contiennent.

De nombreux dérivés d'acylaminophényl ont été décrits dans la littérature. Certains d'entre eux présentent des propriétés inhibitrices de l'acyl CoA-cholestérol-acyltransférase ou ACAT.

C'est le cas, plus particulièrement, des composés décrits dans les brevets EP 242 610, US 4 716 175, EP 344 425, EP 384 320 ou US 4 623 662.

Les dérivés de la présente invention se différencient de ceux décrits dans l'art antérieur non seulement par le fait que ce sont des acylaminophénols comportant au moins trois autres substituants sur ce noyau mais encore par l'intensité et l'originalité de leurs propriétés pharmacologiques et thérapeutiques.

En effet, pour ces dérivés, ont été mises en évidence d'une part leur activité inhibitrice de l'acyl CoA-cholestérol-acyltransférase (ACAT) et d'autre part leur capacité à protéger les lipoprotéines humaines de faible densité assurant le transport du cholestérol (LDL) vis-à-vis des modifications oxydatives induites par le cuivre.

L'activité ACAT responsable de l'estérification intracellulaire du cholestérol libre est présente au niveau des cellules intestinales (entérocytes) et joue un rôle important dans l'absorption intestinale du cholestérol.

Elle est également retrouvée au niveau des cellules musculaires lisses vasculaires et des macrophages et est impliquée dans l'accumulation d'esters de cholestérol au niveau de ces cellules qui contribuent à la formation des lésions vasculaires athéroscléreuses.

Le pouvoir inhibiteur de l'activité ACAT des composés de l'invention leur confère la potentialité d'une part de diminuer les taux plasmatiques de cholestérol en réduisant son absorption intestinale, d'autre part de limiter la progression des lésions vasculaires athéroscléreuses en inhibant l'accumulation d'esters de cholestérol dans la paroi vasculaire.

Les modifications oxydatives des LDL apparaissent par ailleurs constituer un mécanisme important de la formation et de l'extension des lésions vasculaires athéroscléreuses. Aussi les propriétés inhibitrices de la modification oxydative des LDL constituent une deuxième activité thérapeutique tout à fait intéressante.

Cette double activité, des composés de l'invention, qui n'a jamais été montrée pour des composés décrits dans l'art antérieur permet d'envisager leur utilisation comme médicament dans le traitement des différents types de dyslipidémies et de l'athérosclérose avec ses différentes localisations vasculaires périphériques, coronaires et cérébrales.

Plus spécifiquement, la présente invention concerne les composés de formule (I) :

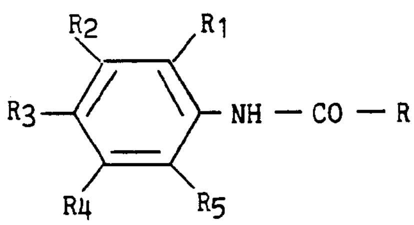

(I)

dans laquelle :

R représente un groupement alkyle ($C_8$-$C_{21}$) linéaire ou ramifié, un groupement 1-[alkyle($C_8$-$C_{21}$)]cycloalk-1-yle($C_3$-$C_6$)linéaire ou ramifié, ou un groupement alcényle ($C_8$-$C_{21}$) linéaire ou ramifié comportant de une à trois double liaisons,

et <u>soit</u>

- $R_3$ représente un groupement hydroxy,
- $R_1$ et $R_2$, différents, représentent un atome d'hydrogène ou un groupement alkyle ($C_1$-$C_6$) linéaire ou ramifié,
- $R_4$ et $R_5$ identiques ou différents représentent un groupement alkyle ($C_1$-$C_6$) linéaire ou ramifié ou un groupement alkoxy ($C_1$-$C_6$) linéaire ou ramifié,

<u>soit</u>

- $R_1$ et $R_4$ représentent simultanément un groupement hydroxy,
- $R_2$, $R_3$ et $R_5$, identiques ou différents représentent un groupement alkyle ($C_1$-$C_6$) linéaire ou ramifié ou un groupement alkoxy ($C_1$-$C_6$) linéaire ou ramifié,

leurs énantiomères, diastéréoisomères et épimères, leurs isomères cis/trans, ainsi que leurs sels d'addition à une base pharmaceutiquement acceptable.

Parmi les bases pharmaceutiquement acceptables, on peut citer à titre non limitatif l'hydroxyde de sodium

ou l'hydroxyde de potassium...

La présente invention concerne également le procédé de préparation des composés de formule (I) caractérisé en ce que l'on utilise comme matière première un composé de formule (II)

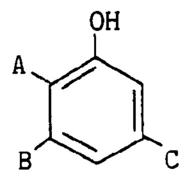

(II)

dans laquelle les substituants A, B ou C identiques ou différents représentent un groupement alkyle ($C_1$-$C_6$) linéaire ou ramifié ou alkoxy ($C_1$-$C_6$) linéaire ou ramifié

que l'on fait réagir :

1

soit en présence de persulfate d'un métal alcalin en présence d'une base,

pour conduire au diphénol de formule (II/a) :

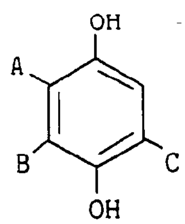

(II/a)

dans laquelle les substituants A, B et C ont la même signification que précédemment,

qui est traité par de l'acide nitrique en milieu acétique, pour conduire à une p.benzoquinone de formule (III) :

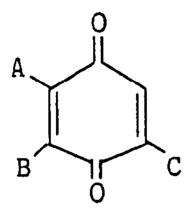

(III)

dans laquelle les substituants A, B et C ont la même signification que précédemment,

qui est ensuite traitée

ou bien

dans le cas où les composés de formule (I) que l'on souhaite obtenir possèdent un groupement $R_3$=OH et dans ce cas A, B et C peuvent être respectivement remplacés par $R_5$, $R_4$ et $R_2$,

en présence d'hydroxylamine en milieu chlorhydrique pour conduire au composé de formule (IV) :

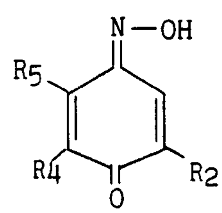

(IV)

dans laquelle :

$R_2$, $R_4$ et $R_5$ ont la même signification que dans la formule (I),

ou bien

dans le cas où les composés de formule (I) que l'on souhaite obtenir possèdent des groupements

$R_1=R_4=OH$ et dans ce cas A, B et C peuvent être respectivement remplacés par $R_2$, $R_3$ et $R_5$,

en présence d'acide chlorhydrique en milieu aqueux puis d'acide nitrique pour conduire au composé de formule (V) :

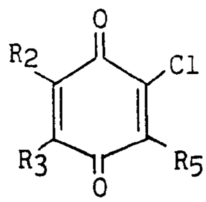

(V)

dans laquelle :

$R_2$, $R_3$ et $R_5$ ont la même signification que dans la formule (I)

que l'on met ensuite en réaction en présence d'azidure de sodium,

pour conduire au composé de formule (VI) :

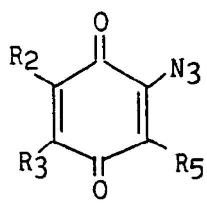

(VI)

dans laquelle $R_2$, $R_3$ et $R_5$ ont la même signification que dans la formule (I),

2

soit en présence de nitrate de sodium et en utilisant le trinitrate de lanthane hexahydraté comme catalyseur en milieu chlorhydrique,

pour conduire à un mélange des composés (VII) et (VIII), que l'on sépare par une technique classique de séparation,

dans le cas où les composés de formule (I) que l'on souhaite obtenir possèdent :

a des groupements $R_1=R_4=OH$ et dans ce cas A, B et C peuvent être respectivement remplacés par $R_2$, $R_3$ et $R_5$

ou

b un groupement $R_3=OH$ et dans ce cas A, B et C peuvent être respectivement remplacés par $R_4$, $R_5$ et $R_1$

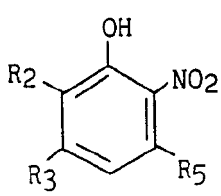

(VII)

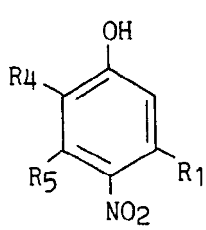

(VIII)

dans lesquelles :

$R_1$, $R_2$, $R_3$, $R_4$ et $R_5$ ont la même signification que dans la formule (I),

dérivé de formule (VII) que l'on met en réaction avec une solution aqueuse de persulfate d'un métal alcalin

en présence d'une base puis que l'on traite par de l'acide sulfurique concentré, pour conduire au composé de formule (IX) :

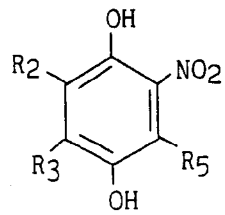

(IX)

dans laquelle :

$R_2$, $R_3$ et $R_5$ ont la même signification que dans la formule (I), dérivés de formules (IV), (VI), (VIII) et (IX) que l'on soumet à une hydrogénation catalytique, pour conduire aux composés de formules (X), (XI) et (XII), que l'on conserve sous atmosphère inerte :

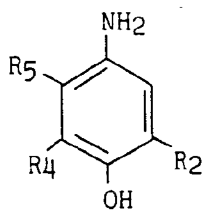

(X)

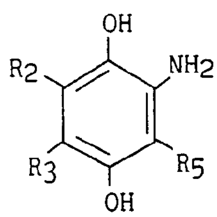

(XI)

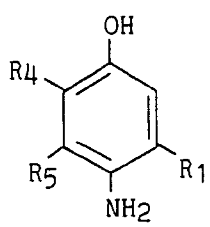

(XII)

dans lesquelles :

$R_1$, $R_2$, $R_3$, $R_4$ et $R_5$ ont la même signification que dans la formule (I), dérivés de formules (X), (XI) et (XII) que l'on fait réagir, sous atmosphère inerte :

ou bien avec un composé de formule (XIII) :

$$R - CO - O - CO - O - CH_2 - CH_3 \qquad (XIII)$$

dans laquelle R a la même signification que dans la formule (I),

ou bien avec un composé de formule (XIV), en présence d'une base organique :

$$R - CO - Cl \qquad (XIV)$$

dans laquelle R a la même signification que dans la formule (I), pour conduire respectivement aux composés de formule (I/a), (I/b) et (I/c) qui constituent l'ensemble des composés de formule (I) :

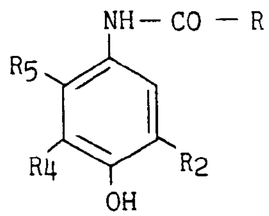

(I/a)

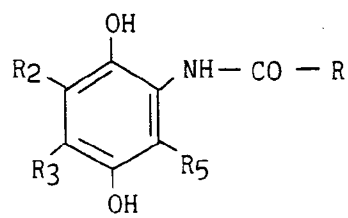

(I/b)

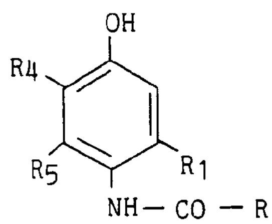

(I/c)

composés de formule (I/a), (I/b), (I/c) que l'on purifie par une technique classique de purification dont on sépare, le cas échéant, les isomères optiques ou cis/trans par des techniques classiques de séparation et que l'on peut transformer en leurs sels d'addition à une base pharmaceutiquement acceptable.

La présente invention a également pour objet les compositions pharmaceutiques renfermant comme principe actif un composé de formule générale (I) ou un de ses sels d'addition à une base pharmaceutiquement acceptable, seul ou en combinaison avec un ou plusieurs excipients ou véhicules inertes non toxiques.

Parmi les compositions pharmaceutiques selon l'invention, on pourra citer celles qui conviennent pour l'administration orale, rectale, parentérale, nasale, les comprimés simples ou dragéifiés, les comprimés sublinguaux, les sachets, les paquets, les gélules, les glossettes, les tablettes, les suppositoires, etc...

La posologie varie selon l'âge et le poids du patient, la nature et la sévérité de l'affection ainsi que la voie d'administration. D'une manière générale, la posologie unitaire s'échelonne entre 50 et 750 mg pour un traitement en 1 à 3 prises par 24 heures.

Les exemples suivants illustrent l'invention mais ne la limitent en aucune façon.

**PREPARATION A :**

La préparation suivante conduit à des intermédiaires utiles dans le procédé de synthèse des composés de l'invention.

2,3,5-triméthyl-6-nitrophénol et 2,3,5-triméthyl-4-nitrophénol

A une solution fortement agitée contenant 300 mmoles de nitrate de sodium et 3 mmoles de trinitrate de lanthane hexahydraté dans 300 ml d'eau et 180 ml d'acide chlorhydrique à 37 %, est ajoutée, goutte à goutte, une solution contenant 300 mmoles de 2,3,5-triméthylphénol dans 900 ml d'éther éthylique en maintenant la température vers 6°C. Après cette addition, le mélange réactionnel est agité à 20°C pendant 4 heures, puis la phase aqueuse est éliminée après décantation.

La phase organique est lavée par de l'eau, séchée et évaporée. Le résidu est chromatographié sur colonne

de silice (solvant d'élution : hexane/acétate d'éthyle : 85/15) et conduit aux deux produits attendus.
2,3,5-triméthyl-6-nitrophénol
Rendement : 38 %
Point de fusion : 77-78°C
Microanalyse élémentaire :

| | C % | H % | N % |
|---|---|---|---|
| calculé | 59,66 | 6,12 | 7,73 |
| trouvé | 60,07 | 6,34 | 7,77 |

2,3,5-triméthyl-4-nitrophénol
Rendement : 18 %
Point de fusion : 80-81°C
Microanalyse élémentaire :

| | C % | H % | N % |
|---|---|---|---|
| calculé | 59,66 | 6,12 | 7,73 |
| trouvé | 59,58 | 6,17 | 8,06 |

**EXEMPLE 1: 4-(9-Octadécènamido)-2,3,5-triméthylphénol, isomère cis**

STADE A : 2,3,5-triméthyl-4-aminophénol

Le produit attendu est obtenu par hydrogénation catalytique de 8 mmoles de 2,3,5-triméthyl-4-nitrophénol obtenu dans la préparation A en solution dans 20 ml de tétrahydrofurane anhydre, à basse pression, en présence de 50 mg d'oxyde de platine, à 20°C.

STADE B : 4-(9-Octadécènamido)-2,3,5-triméthylphénol, isomère cis

A la solution précédente est ajoutée, à 5°C, sous atmosphère d'azote, une suspension de carbethoxy oléate (préparée par réaction, à -10°C, en 15 minutes, sous atmosphère d'azote, de 8 mmoles d'acide oléique avec 8 mmoles de chloroformiate d'éthyle en présence de 8 mmoles de triéthylamine dans 20 ml de tétrahydrofurane anhydre).

Le mélange réactionnel est agité une heure à 20°C. Le solvant est alors évaporé et le résidu est repris par 50 ml d'acétate d'éthyle. La phase organique est lavée par de l'eau, séchée et évaporée.

Le produit attendu est obtenu par recristallisation dans de l'éther isopropylique.
Rendement : 72 %

Le stade B de cet exemple constitue la méthode A de notre procédé de synthèse.

**EXEMPLE 2 : 2,3,6-Triméthyl-4-nonanamidophénol**

STADE A : 2,3,5-Triméthylhydroquinone

Une solution contenant 100 mmoles de persulfate de potassium dans un litre d'eau est additionnée goutte à goutte sous agitation, à 20°C, à une solution contenant 100 mmoles de 2,3,5-triméthylphénol dans 100 ml d'une solution d'hydroxyde de sodium à 10 %. Après 20 heures d'agitation, le milieu réactionnel est neutralisé par de l'acide sulfurique concentré et le solide formé est filtré. La solution aqueuse restante est acidifiée par addition lente de 220 ml d'acide sulfurique concentrée et chauffée 30 minutes à 80°C. L'huile formée est décantée et extraite par de l'éther éthylique.

Le produit attendu est obtenu après séchage de la phase éthérée, évaporation et recristallisation dans de l'acétate d'éthyle.
Rendement : 93 %
Point de fusion : 172-174°C

STADE B : 2,3,5-Triméthyl-p.benzoquinone

80 mmoles du produit obtenu au stade précédent en solution dans 75 ml d'acide acétique sont traitées, à 16°C, par addition, goutte à goutte d'une solution contenant 3,8 ml d'acide nitrique dans 15 ml d'acide acétique.

Après une heure d'agitation à 20°C, le milieu réactionnel est versé sur 350 ml d'eau glacée. Le produit attendu est obtenu sous forme de précipité qui est filtré, lavé par de l'eau glacée et séché.

Rendement : 95 %
Point de fusion : 29-30°C
Microanalyse élémentaire :

| | C % | H % |
|---|---|---|
| calculée | 71,98 | 6,71 |
| trouvée | 71,76 | 6,65 |

STADE C : 2,3,6-Triméthyl-4-hydroxyimino-p.benzoquinone

33 mmoles du produit obtenu au stade précédent et 33 mmoles de chlorhydrate d'hydroxylamine sont portées au reflux dans 340 ml d'acide chlorhydrique 2N pendant 6 heures. Le produit attendu est obtenu par filtration du précipité après refroidissement, lavage à l'eau puis au cyclohexane.

Rendement : 85 %
Point de fusion : 185-186°C
Microanalyse élémentaire :

| | C % | H % | N % |
|---|---|---|---|
| calculé | 65,44 | 6,71 | 8,48 |
| trouvé | 65,56 | 6,78 | 8,66 |

STADE D : 2,3,6-Triméthyl-4-aminophénol

STADE E : 2,3,6-Triméthyl-4-nonanamidophénol

Les stades D et E sont identiques aux stades A et B de l'exemple 1.

**EXEMPLE 3 : 2,3-Diméthoxy-6-méthyl-4-nonanamidophénol**

STADE A : 2,3-Diméthoxy-5-méthyl-p.benzoquinone

Le produit attendu est obtenu en utilisant le même mode opératoire que celui décrit aux stades A et B de l'exemple 2.

STADE C : 2,3-Diméthoxy-6-méthyl-4-hydroxyimino-p.benzoquinone

Un mélange contenant 88 mmoles du produit obtenu au stade précédent, 158 mmoles de chlorhydrate d'hydroxylamine et 11 mmoles d'acétate de sodium dans 160 ml de méthanol est porté au reflux pendant 3 heures. Après évaporation du méthanol, le produit attendu est obtenu, à 0°C, par filtration du précipité formé.

Rendement : 68 %
Point de fusion : 141-142°C
Microanalyse élémentaire :

| | C % | H % | N % |
|---|---|---|---|
| calculé | 54,82 | 5,62 | 7,10 |
| trouvé | 54,45 | 5,57 | 7,21 |

STADE D : 2,3-Diméthoxy-6-méthyl-4-aminophénol

STADE E : 2,6-Diméthoxy-6-méthyl-4-nonanamidophénol

Les stades D et E sont identiques aux stades A et B de l'exemple 1.

**EXEMPLE 4 : 2,3,6-Triméthyl-4-(2-méthylundécanamido)phénol**

STADES A, B et C : 2,3,6-Triméthyl-4-aminophénol

Les stades A, B et C sont identiques aux stades A, B et C décrits dans l'exemple 3.

STADE D : 2,3,6-Triméthyl-4-(2-méthylundécanamido)phénol

A la solution précédente sont ajoutées successivement 8 mmoles de triéthylamine et 8 mmoles de chlorure de l'acide 2-méthylundécanoïque. Après agitation à 20°C pendant une nuit, le solvant est évaporé et le résidu huileux est dissous dans 75 ml d'éther éthylique. La phase éthérée est lavée par de l'eau, séchée et concentrée sous vide. Le produit attendu est obtenu par recristallisation du résidu dans l'éther isopropylique.
Rendement : 64 %

Le stade D de cet exemple constitue la méthode B de notre procédé de synthèse.

Les exemples 5 à 35 ainsi que 57 et 58 ont été préparés selon l'une des méthodes A ou B décrites dans les exemples 1 ou 4 et en utilisant des dérivés de 4-aminophénol préparés selon l'un quelconque des procédés décrits dans les exemples 1 ou 2.

Les méthodes A ou B utilisées sont précisées dans le tableaux 1, ainsi que les rendements, points de fusion et résultats de microanalyse élémentaire.

**EXEMPLE 5 : 2,3,6-Triméthyl-4-décanamidophénol**

**EXEMPLE 6 : 2,3,6-Triméthyl-4-undécanamidophénol**

**EXEMPLE 7 : 2,3,6-Triméthyl-4-dodécanamidophénol**

**EXEMPLE 8 : 2,3,6-Triméthyl-4-tridécanamidophénol**

**EXEMPLE 9 : 2,3,6-Triméthyl-4-tétradécanamidophénol**

**EXEMPLE 10 : 2,3,6-Triméthyl-4-hexadécanamidophénol**

**EXEMPLE 11 : 2,3,6-Triméthyl-4-octadécanamidophénol**

**EXEMPLE 12 : 2,3,6-Triméthyl-4-(2-méthyldécanamido)phénol**

**EXEMPLE 13 : 2,3,6-Triméthyl-4-(2,2-diméthyldécanamido)phénol**

**EXEMPLE 14 : 2,3,6-Triméthyl-4-(2,2-diméthylundécanamido)phénol**

**EXEMPLE 15 : 2,3,6-Triméthyl-4-(2,2-diméthyldodécanamido)phénol**

**EXEMPLE 16 : 2,3,6-Triméthyl-4-(2,2-diméthyltridécanamido)phénol**

**EXEMPLE 17 : 2,3,6-Triméthyl-4-(2,2-diméthyloctadécanamido)phénol**

**EXEMPLE 18 : 2,3,6-Triméthyl-4-(9-octadécènamido)phénol, isomère cis**

**EXEMPLE 19 : 2,3,6-Triméthyl-4-(9-octadécènamido)phénol, isomère trans**

**EXEMPLE 20 : 2,3,6-Triméthyl-4-(2-méthyl-9-octadécènamido)phénol, isomère cis**

**EXEMPLE 21 : 2,3,6-Triméthyl-4-(9,12-octadécadiènamido)phénol, isomère cis, cis**

**EXEMPLE 22 : 2,3,6-Triméthyl-4-(9,12,15-octadécatriènamido)phénol**

**EXEMPLE 23 : 2,3,6-Triméthyl-4-(6,9,12-octadécatriènamido)phénol**

**EXEMPLE 24 : 2,3,5-Triméthyl-4-undécanamidophénol**

**EXEMPLE 25 : 2,3,5-Triméthyl-4-tétradécanamidophénol**

**EXEMPLE 26 : 2,3,5-Triméthyl-4-octadécanamidophénol**

**EXEMPLE 27 : 2,3,5-Triméthyl-4-(9,12-octadécadiénamido)phénol, isomère cis, cis**

**EXEMPLE 28 : 2,3-Diméthoxy-6-méthyl-4-décanamidophénol**

**EXEMPLE 29 : 2,3-Diméthoxy-6-méthyl-4-undécanamidophénol**

**EXEMPLE 30 : 2,3-Diméthoxy-6-méthyl-4-dodécanamidophénol**

**EXEMPLE 31 : 2,3-Diméthoxy-6-méthyl-4-tridécanamidophénol**

**EXEMPLE 32 : 2,3-Diméthoxy-6-méthyl-4-tétradécanamidophénol**

**EXEMPLE 33 : 2,3-Diméthoxy-6-méthyl-4-hexadécanamidophénol**

**EXEMPLE 34 : 2,3-Diméthoxy-6-méthyl-4-octadécanamidophénol**

**EXEMPLE 35 : 2,3-Diméthoxy-6-méthyl-4-(9-octadécènamido)phénol, isomère cis**

**EXEMPLE 36 : 3,5,6-Triméthyl-2-nonanamidohydroquinone**

STADE A : 3,5,6-Triméthyl-2-nitrohydroquinone

Une solution contenant 97 mmoles de persulfate de potassium dans 1100 ml d'eau est additionnée goutte à goutte, sous agitation, à 20°C, à une suspension contenant 97 mmoles de 2,3,5-triméthyl-6-nitrophénol (obtenu dans la préparation A) dans 195 ml d'une solution d'hydroxyde de sodium à 10 %.

Après 20 heures d'agitation, le milieu réactionnel est neutralisé par de l'acide sulfurique concentré. Le solide formé est filtré. La solution aqueuse restante est acidifiée par addition de 200 ml d'acide sulfurique concentré et chauffée 30 minutes à 70°C. L'huile formée est décantée et extraite par de l'éther éthylique.

Le produit attendu est obtenu après séchage de la phase éthérée, évaporation et recristallisation dans un mélange acétate d'éthyle/hexane.

Rendement : 85 %
Point de fusion : 105-106°C
Microanalyse élémentaire :

| | C % | H % | N % |
|---|---|---|---|
| calculé | 54,82 | 5,62 | 7,10 |
| trouvé | 54,68 | 5,62 | 7,30 |

STADE B : 2-Amino-3,5,6-triméthylhydroquinone

L'hydrogénation catalytique du produit obtenu au stade A est réalisée selon le même mode opératoire que celui décrit au stade A de l'exemple 1.

STADE C : 3,5,6-Triméthyl-2-nonanamidohydroquinone

Le mode opératoire utilisé est la méthode A décrite au stade B de l'exemple 1.

Les exemples suivants ont été synthétisés en suivant le même mode opératoire que celui décrit aux deux premiers stades de l'exemple 36 et en utilisant au stade C l'une des méthodes A ou B comme il est précisé dans le tableau II. Le tableau II contient les rendements, points de fusion et résultats de microanalyse élémentaire des produits obtenus.

**EXEMPLE 37 : 3,5,6-Triméthyl-2-décanamidohydroquinone**

**EXEMPLE 38 : 3,5,6-Triméthyl-2-undécanamidohydroquinone**

**EXEMPLE 39 : 3,5,6-Triméthyl-2-dodécanamidohydroquinone**

**EXEMPLE 40 : 3,5,6-Triméthyl-2-tridécanamidohydroquinone**

**EXEMPLE 41 : 3,5,6-Triméthyl-2-tétradécanamidohydroquinone**

**EXEMPLE 42 : 3,5,6-Triméthyl-2-hexadécanamidohydroquinone**

**EXEMPLE 43 : 3,5,6-Triméthyl-2-octadécanamidohydroquinone**

**EXEMPLE 44 : 3,5,6-Triméthyl-2-(2,2,diméthyloctadécanamido)hydroquinone**

**EXEMPLE 45 : 3,5,6-Triméthyl-2-(9-octadécènamido)hydroquinone, isomère cis**

**EXEMPLE 46 : 3,5,6-Triméthyl-2-(9-octadécènamido)hydroquinone, isomère trans**

**EXEMPLE 47 : 3,5,6-Triméthyl-2-(9,12-octadécadiènamido)hydroquinone**

**EXEMPLE 48 : 3,5,6-Triméthyl-2-(9,12,15,octadécatriènamido)hydroquinone**

**EXEMPLE 49 : 5,6-Diméthoxy-3-méthyl-2-dodécanamidohydroquinone**

STADE A : 2,3-Diméthoxy-6-méthyl-p.benzoquinone

Ce stade est identique au stade A de l'exemple 2.

STADE B : 2,3-Diméthoxy-5-chloro-6-méthyl-p.benzoquinone

Une suspension contenant 270 mmoles du produit obtenu au stade A dans 300 ml d'acide chlorhydrique à 37 % et 100 ml d'eau est agitée, pendant trois heures à température ambiante.

Cette suspension est ensuite versée sur un litre d'eau glacée et extraite par de l'éther éthylique. Les phases éthérées sont lavées par une solution aqueuse saturée en chlorure de sodium jusqu'à neutralité, et évaporées. Le résidu est repris par un litre d'éther refroidi à 0°C et traité par 35 ml d'acide nitrique pendant trois heures. Après une heure d'agitation à 10°C, le mélange est versé sur un litre d'eau glacée et extrait par de l'éther éthylique. La phase aqueuse est lavée par 100 ml d'une solution saturée en chlorure de sodium, séchée et concentrée.

Le produit attendu est obtenu après recristallisation du résidu dans le cyclohexane.

Rendement : 70 %
Point de fusion : 66-68°C
Microanalyse élémentaire :

| | C % | H % | N % |
|---|---|---|---|
| calculée | 49,90 | 4,19 | 16,37 |
| trouvée | 49,74 | 4,13 | 16,24 |

STADE C : 2,3-Diméthoxy-5-azido-6-méthyl-p.benzoquinone

Une solution contenant 37 mmoles d'azidure de sodium dans 30 ml d'eau est ajoutée à une solution contenant 18,5 mmoles du produit obtenu au stade précédent dans 80 ml d'éthanol à 95 %. Après une heure d'agitation à température ambiante, le mélange réactionnel est versé sur 300 ml d'eau glacée et extrait par de l'éther. Les phases éthérées sont séchées et concentrées.

Le produit attendu est obtenu par recristallisation du résidu dans du cyclohexane.

Rendement : 87 %

Point de fusion : 48-50°C

Microanalyse élémentaire :

| | C % | H % | N % |
|---|---|---|---|
| calculée | 48,42 | 4,04 | 18,83 |
| trouvée | 48,31 | 4,10 | 18,64 |

STADE D : 5,6-Diméthoxy-3-méthyl-2-dodécanamidohydroquinone

Le produit attendu est obtenu en procédant comme au stade B de l'exemple 1 mais en remplaçant l'acide oléique par l'acide laurique.

Rendement : 84 %

Les exemples suivants ont été synthétisés en suivant le même mode opératoire que celui décrit aux trois premiers stades de l'exemple 48 et en utilisant au stade D l'une des méthodes A ou B comme il est précisé dans le tableau II. Le tableau II contient également les rendements, points de fusion et résultats de microanalyse élémentaire des produits obtenus.

**EXEMPLE 50 : 5,6-Diméthoxy-3-méthyl-2-décanamidohydroquinone**

**EXEMPLE 51 : 5,6-Diméthoxy-3-méthyl-2-undécanamidohydroquinone**

**EXEMPLE 52 : 5,6-Diméthoxy-3-méthyl-2-tridécanamidohydroquinone**

**EXEMPLE 53 : 5,6-Diméthoxy-3-méthyl-2-tétradécamidohydroquinone**

**EXEMPLE 54 : 5,6-Diméthoxy-3-méthyl-2-hexadécamidohydroquinone**

**EXEMPLE 55 : 5,6-Diméthoxy-3-méthyl-2-octadécanamidohydroquinone**

**EXEMPLE 56 : 5,6-Diméthoxy-3-méthyl-2-(9-octadécènamido)hydroquinone, isomère cis**

**EXEMPLE 57 : 2,3,6-Triméthyl-4-[(1-décyl-cyclopent-1-yl)carboxamido]phénol**

**EXEMPLE 58 : 2,3,6-Triméthyl-4-(2-méthyl-9,12-octadécadiènamido)phénol, isomère cis, cis**

## TABLEAU I

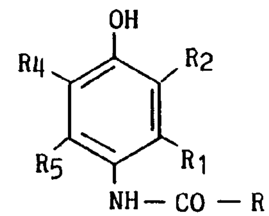

| N° Exemple | $R_1$ | $R_2$ | $R_4$ | $R_5$ | R | Méthode | Rendement (%) | Point de fusion (°C) | Microanalyse élémentaire | | | |
|---|---|---|---|---|---|---|---|---|---|---|---|---|
| | | | | | | | | | | C % | H % | N % |
| 1 | $CH_3$ | H | $CH_3$ | $CH_3$ | $CH_3-(CH_2)_7-CH=CH-(CH_2)_7-$ (cis) | A | 72 | 107-108 | calculée<br>trouvée | 78,02<br>77,99 | 10,91<br>10,71 | 3,37<br>3,40 |
| 2 | H | $CH_3$ | $CH_3$ | $CH_3$ | $CH_3-(CH_2)_7-$ | A | 69 | 143-145 | calculée<br>trouvée | 74,18<br>73,84 | 10,03<br>10,03 | 4,81<br>4,74 |
| 3 | H | $CH_3$ | $CH_3O$ | $CH_3O$ | $CH_3-(CH_2)_7-$ | A | 63 | 61-62 | calculée<br>trouvée | 66,84<br>66,61 | 9,04<br>8,97 | 4,33<br>4,20 |
| 4 | H | $CH_3$ | $CH_3$ | $CH_3$ | $CH_3-(CH_2)_8-CHCH_3-$ | B | 64 | 149-151 | calculée<br>trouvée | 75,63<br>75,10 | 10,58<br>10,51 | 4,20<br>4,24 |
| 5 | H | $CH_3$ | $CH_3$ | $CH_3$ | $CH_3-(CH_2)_8-$ | A | 77 | 136-138 | calculée<br>trouvée | 74,71<br>74,44 | 10,23<br>10,13 | 4,59<br>4,79 |
| 6 | H | $CH_3$ | $CH_3$ | $CH_3$ | $CH_3-(CH_2)_9-$ | A | 82 | 142-144 | calculée<br>trouvée | 75,19<br>75,28 | 10,41<br>10,31 | 4,38<br>4,32 |
| 7 | H | $CH_3$ | $CH_3$ | $CH_3$ | $CH_3-(CH_2)_{10}-$ | A | 80 | 137-140 | calculée<br>trouvée | 75,63<br>75,51 | 10,58<br>10,95 | 4,20<br>4,23 |
| 8 | H | $CH_3$ | $CH_3$ | $CH_3$ | $CH_3-(CH_2)_{11}-$ | A | 80 | 140-141 | calculée<br>trouvée | 76,03<br>76,12 | 10,73<br>10,64 | 4,03<br>4,05 |
| 9 | H | $CH_3$ | $CH_3$ | $CH_3$ | $CH_3-(CH_2)_{12}-$ | A | 77 | 142-144 | calculée<br>trouvée | 76,40<br>76,05 | 10,87<br>10,97 | 3,87<br>4,13 |

TABLEAU I (suite 1)

| N° Exemple | $R_1$ | $R_2$ | $R_4$ | $R_5$ | R | Méthode | Rendement (%) | Point de fusion (°C) | Microanalyse élémentaire | C % | H % | N % |
|---|---|---|---|---|---|---|---|---|---|---|---|---|
| 10 | H | $CH_3$ | $CH_3$ | $CH_3$ | $CH_3-(CH_2)_{14}-$ | A | 79 | 135-138 | calculée<br>trouvée | 77,07<br>76,85 | 11,12<br>10,97 | 3,59<br>3,51 |
| 11 | H | $CH_3$ | $CH_3$ | $CH_3$ | $CH_3-(CH_2)_{16}-$ | A | 80 | 138-140 | calculée<br>trouvée | 77,64<br>77,31 | 11,34<br>11,40 | 3,35<br>3,33 |
| 12 | H | $CH_3$ | $CH_3$ | $CH_3$ | $CH_3-(CH_2)_7-CHCH_3-$ | B | 68 | 148-150 | calculée<br>trouvée | 75,19<br>74,97 | 10,41<br>10,40 | 4,38<br>4,71 |
| 13 | H | $CH_3$ | $CH_3$ | $CH_3$ | $CH_3-(CH_2)_7-C(CH_3)_2-$ | B | 58 | 68-71 | calculée<br>trouvée | 75,63<br>75,63 | 10,58<br>10,49 | 4,20<br>4,30 |
| 14 | H | $CH_3$ | $CH_3$ | $CH_3$ | $CH_3-(CH_2)_8-C(CH_3)_2-$ | B | 63 | 87-89 | calculée<br>trouvée | 76,03<br>75,67 | 10,73<br>10,34 | 4,03<br>4,50 |
| 15 | H | $CH_3$ | $CH_3$ | $CH_3$ | $CH_3-(CH_2)_9-C(CH_3)_2-$ | B | 60 | 75-76 | calculée<br>trouvée | 76,40<br>76,30 | 10,87<br>10,77 | 3,87<br>4,11 |
| 16 | H | $CH_3$ | $CH_3$ | $CH_3$ | $CH_3-(CH_2)_{10}-C(CH_3)_2-$ | B | 58 | 80-82 | calculée<br>trouvée | 76,75<br>76,55 | 11,00<br>11,40 | 3,73<br>4,00 |
| 17 | H | $CH_3$ | $CH_3$ | $CH_3$ | $CH_3-(CH_2)_{15}-C(CH_3)_2-$ | B | 64 | 79-81 | calculée<br>trouvée | 78,15<br>78,41 | 11,53<br>11,52 | 3,14<br>3,02 |
| 18 | H | $CH_3$ | $CH_3$ | $CH_3$ | $CH_3-(CH_2)_7-CH=CH(CH_2)_7-$ (cis) | A | 57 | 125-127 | calculée<br>trouvée | 78,02<br>77,97 | 10,91<br>10,99 | 3,37<br>3,64 |
| 19 | H | $CH_3$ | $CH_3$ | $CH_3$ | $CH_3-(CH_2)_7-CH=CH-(CH_2)_7-$ (trans) | A | 59 | 127-129 | calculée<br>trouvée | 78,02<br>78,15 | 10,91<br>10,94 | 3,37<br>3,31 |

**TABLEAU I (suite 2)**

| N° Exemple | $R_1$ | $R_2$ | $R_4$ | $R_5$ | R | Méthode | Rendement (%) | Point de fusion (°C) | Microanalyse élémentaire | C % | H % | N % |
|---|---|---|---|---|---|---|---|---|---|---|---|---|
| 20 | H | $CH_3$ | $CH_3$ | $CH_3$ | $CH_3-(CH_2)_7-CH=CH-(CH_2)_6-CH(CH_3)-$ (cis) | B | 55 | 125-127 | calculée<br>trouvée | 78,27<br>78,04 | 11,03<br>10,95 | 3,26<br>3,20 |
| 21 | H | $CH_3$ | $CH_3$ | $CH_3$ | $CH_3-(CH_2)_3-(CH_2-CH=CH)_2-(CH_2)_7-$ (cis, cis) | A | 68 | 130-132 | calculée<br>trouvée | 78,40<br>78,11 | 10,48<br>10,45 | 3,39<br>3,67 |
| 22 | H | $CH_3$ | $CH_3$ | $CH_3$ | $CH_3-(CH_2-CH=CH)_3-(CH_2)_7-$ | A | 66 | 117-118 | calculée<br>trouvée | 78,78<br>78,91 | 10,04<br>10,23 | 3,40<br>3,55 |
| 23 | H | $CH_3$ | $CH_3$ | $CH_3$ | $CH_3-(CH_2)_3-(CH_2-CH=CH)_3-(CH_2)_4-$ | A | 71 | 106-109 | calculée<br>trouvée | 78,78<br>77,93 | 10,04<br>9,88 | 3,40<br>3,91 |
| 24 | $CH_3$ | H | $CH_3$ | $CH_3$ | $CH_3-(CH_2)_9-$ | A | 67 | 118-120 | calculée<br>trouvée | 75,19<br>75,10 | 10,49<br>10,42 | 4,38<br>4,56 |
| 25 | $CH_3$ | H | $CH_3$ | $CH_3$ | $CH_3-(CH_2)_{12}-$ | A | 74 | 122-124 | calculée<br>trouvée | 76,40<br>76,39 | 10,87<br>10,97 | 3,87<br>3,77 |
| 26 | $CH_3$ | H | $CH_3$ | $CH_3$ | $CH_3-(CH_2)_{16}-$ | A | 72 | 123-125 | calculée<br>trouvée | 77,64<br>77,68 | 11,34<br>11,52 | 3,35<br>3,40 |
| 27 | $CH_3$ | H | $CH_3$ | $CH_3$ | $CH_3-(CH_2)_3-(CH_2-CH=CH)_2-(CH_2)_7-$ (cis, cis) | A | 59 | 102-104 | calculée<br>trouvée | 78,40<br>77,81 | 10,48<br>10,42 | 3,39<br>3,29 |
| 28 | H | $CH_3$ | $CH_3O$ | $CH_3O$ | $CH_3-(CH_2)_8-$ | A | 64 | 72-73 | calculée<br>trouvée | 67,63<br>67,27 | 9,26<br>9,24 | 4,15<br>4,11 |

**TABLEAU I** (suite 3)

| N° Exemple | R1 | R2 | R4 | R5 | R | Méthode | Rendement (%) | Point de fusion (°C) | Microanalyse élémentaire | C % | H % | N % |
|---|---|---|---|---|---|---|---|---|---|---|---|---|
| 29 | H | $CH_3$ | $CH_3O$ | $CH_3O$ | $CH_3-(CH_2)_9-$ | A | 68 | 73-75 | calculée<br>trouvée | 68,34<br>68,30 | 9,46<br>9,37 | 3,98<br>4,13 |
| 30 | H | $CH_3$ | $CH_3O$ | $CH_3O$ | $CH_3-(CH_2)_{10}-$ | A | 68 | 82-83 | calculée<br>trouvée | 69,01<br>68,65 | 9,65<br>9,65 | 3,83<br>3,91 |
| 31 | H | $CH_3$ | $CH_3O$ | $CH_3O$ | $CH_3-(CH_2)_{11}-$ | A | 65 | 76-78 | calculée<br>trouvée | 69,62<br>69,41 | 9,83<br>9,83 | 3,69<br>3,87 |
| 32 | H | $CH_3$ | $CH_3O$ | $CH_3O$ | $CH_3-(CH_2)_{12}-$ | A | 62 | 87-90 | calculée<br>trouvée | 70,19<br>69,45 | 9,99<br>9,84 | 3,56<br>3,77 |
| 33 | H | $CH_3$ | $CH_3O$ | $CH_3O$ | $CH_3-(CH_2)_{14}-$ | A | 69 | 93-95 | calculée<br>trouvée | 71,22<br>70,87 | 10,28<br>10,06 | 3,32<br>3,35 |
| 34 | H | $CH_3$ | $CH_3O$ | $CH_3O$ | $CH_3-(CH_2)_{16}-$ | A | 75 | 97-98 | calculée<br>trouvée | 72,12<br>71,78 | 10,54<br>10,53 | 3,11<br>3,14 |
| 35 | H | $CH_3$ | $CH_3O$ | $CH_3O$ | $CH_3-(CH_2)_7-CH=CH-(CH_2)_7-$<br>(cis) | A | 78 | 54-56 | calculée<br>trouvée | 72,44<br>72,28 | 10,13<br>9,95 | 3,13<br>3,21 |
| 57 | H | $CH_3$ | $CH_3$ | $CH_3$ | $CH_3-(CH_2)_9$ 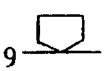 | B | 53 | 99-101 | calculée<br>trouvée | 77,47<br>77,33 | 10,66<br>11,05 | 3,61<br>3,79 |
| 58 | H | $CH_3$ | $CH_3$ | $CH_3$ | $CH_3-(CH_2)_4-CH=CH-CH_2-CH=CH$<br>$-CH-(CH_2)_6$<br>$CH_3$ | A | 68 | 120-122 | calculée<br>trouvée | 78,64<br>78,48 | 10,61<br>10,76 | 3,28<br>3,47 |

## TABLEAU II

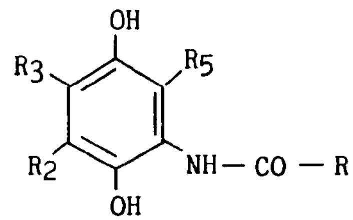

| N° Exemple | $R_2$ | $R_3$ | $R_5$ | R | Méthode | Rendement (%) | Point de fusion (°C) | Microanalyse élémentaire | | | |
|---|---|---|---|---|---|---|---|---|---|---|---|
| | | | | | | | | | C % | H % | N % |
| 36 | $CH_3$ | $CH_3$ | $CH_3$ | $CH_3-(CH_2)_7-$ | A | 77 | 174-178 | calculée<br>trouvée | 70,32<br>70,71 | 9,51<br>9,56 | 4,56<br>4,62 |
| 37 | $CH_3$ | $CH_3$ | $CH_3$ | $CH_3-(CH_2)_8-$ | A | 72 | 165-167 | calculée<br>trouvée | 70,99<br>70,70 | 9,72<br>9,65 | 4,36<br>4,30 |
| 38 | $CH_3$ | $CH_3$ | $CH_3$ | $CH_3-(CH_2)_9-$ | A | 80 | 157-159 | calculée<br>trouvée | 71,60<br>71,66 | 9,91<br>9,95 | 4,17<br>4,34 |
| 39 | $CH_3$ | $CH_3$ | $CH_3$ | $CH_3-(CH_2)_{10}-$ | A | 82 | 159-162 | calculée<br>trouvée | 72,17<br>71,81 | 10,09<br>9,99 | 4,01<br>4,07 |
| 40 | $CH_3$ | $CH_3$ | $CH_3$ | $CH_3-(CH_2)_{11}-$ | A | 78 | 155-157 | calculée<br>trouvée | 72,69<br>73,21 | 10,26<br>10,23 | 3,85<br>3,74 |
| 41 | $CH_3$ | $CH_3$ | $CH_3$ | $CH_3-(CH_2)_{12}-$ | A | 84 | 162-163 | calculée<br>trouvée | 73,17<br>73,25 | 10,41<br>10,76 | 3,71<br>3,77 |

TABLEAU II (suite 1)

| N° Exemple | $R_2$ | $R_3$ | $R_5$ | R | Méthode | Rendement (%) | Point de fusion (°C) | Microanalyse élémentaire | | | |
|---|---|---|---|---|---|---|---|---|---|---|---|
| | | | | | | | | | C % | H % | N % |
| 42 | $CH_3$ | $CH_3$ | $CH_3$ | $CH_3-(CH_2)_{14}-$ | A | 82 | 158-160 | calculée<br>trouvée | 74,03<br>73,84 | 10,69<br>10,66 | 3,45<br>3,41 |
| 43 | $CH_3$ | $CH_3$ | $CH_3$ | $CH_3-(CH_2)_{16}-$ | A | 80 | 153-155 | calculée<br>trouvée | 74,78<br>74,85 | 10,92<br>11,08 | 3,23<br>3,21 |
| 44 | $CH_3$ | $CH_3$ | $CH_3$ | $CH_3-(CH_2)_{15}-C(CH_3)_2-$ | B | 66 | 108-110 | calculée<br>trouvée | 75,44<br>75,27 | 11,13<br>11,19 | 3,03<br>2,97 |
| 45 | $CH_3$ | $CH_3$ | $CH_3$ | $CH_3-(CH_2)_7-CH=CH-(CH_2)_7-$ (cis) | A | 76 | 155-158 | calculée<br>trouvée | 75,13<br>75,19 | 10,51<br>10,49 | 3,24<br>3,01 |
| 46 | $CH_3$ | $CH_3$ | $CH_3$ | $CH_3-(CH_2)_7-CH=CH-(CH_2)_7-$ (trans) | A | 81 | 178-180 | calculée<br>trouvée | 75,13<br>75,55 | 10,51<br>10,58 | 3,24<br>3,18 |
| 47 | $CH_3$ | $CH_3$ | $CH_3$ | $CH_3-(CH_2)_3-(CH_2-CH=CH)_2-(CH_2)_7-$ (cis, cis) | A | 71 | 162-164 | calculée<br>trouvée | 75,48<br>75,56 | 10,09<br>9,94 | 3,26<br>3,00 |
| 48 | $CH_3$ | $CH_3$ | $CH_3$ | $CH_3-(CH_2-CH=CH)_3-(CH_2)_7-$ | A | 68 | 132-134 | calculée<br>trouvée | 75,84<br>76,17 | 9,66<br>9,73 | 3,28<br>3,28 |
| 49 | $CH_3O$ | $CH_3O$ | $CH_3$ | $CH_3-(CH_2)_{10}-$ | A | 84 | 86-89 | calculée<br>trouvée | 66,11<br>65,91 | 9,25<br>9,21 | 3,67<br>3,50 |

TABLEAU II (suite 2)

| N° Exemple | $R_2$ | $R_3$ | $R_5$ | R | Méthode | Rendement (%) | Point de fusion (°C) | Microanalyse élémentaire | | | |
|---|---|---|---|---|---|---|---|---|---|---|---|
| | | | | | | | | | C % | H % | N % |
| 50 | $CH_3O$ | $CH_3O$ | $CH_3$ | $CH_3-(CH_2)_8-$ | A | 80 | 90-92 | calculée<br>trouvée | 64,56<br>64,58 | 8,84<br>8,87 | 3,96<br>3,98 |
| 51 | $CH_3O$ | $CH_3O$ | $CH_3$ | $CH_3-(CH_2)_9-$ | A | 76 | 95-96 | calculée<br>trouvée | 65,37<br>65,24 | 9,05<br>8,99 | 3,81<br>3,77 |
| 52 | $CH_3O$ | $CH_3O$ | $CH_3$ | $CH_3-(CH_2)_{11}-$ | A | 82 | 99-102 | calculée<br>trouvée | 66,81<br>66,81 | 9,43<br>8,99 | 3,54<br>3,38 |
| 53 | $CH_3O$ | $CH_3O$ | $CH_3$ | $CH_3-(CH_2)_{12}-$ | A | 84 | 101-103 | calculée<br>trouvée | 67,45<br>67,29 | 9,60<br>9,63 | 3,42<br>3,64 |
| 54 | $CH_3O$ | $CH_3O$ | $CH_3$ | $CH_3-(CH_2)_{14}-$ | A | 81 | 107-109 | calculée<br>trouvée | 68,62<br>68,81 | 9,90<br>9,90 | 3,20<br>3,31 |
| 55 | $CH_3O$ | $CH_3O$ | $CH_3$ | $CH_3-(CH_2)_{16}-$ | A | 79 | 108-110 | calculée<br>trouvée | 69,64<br>69,79 | 10,17<br>10,30 | 3,01<br>2,95 |
| 56 | $CH_3O$ | $CH_3O$ | $CH_3$ | $CH_3-(CH_2)_7-CH=CH-(CH_2)_7-$ (cis) | A | 68 | 85-87 | calculée<br>trouvée | 69,94<br>70,33 | 9,78<br>9,90 | 3,02<br>3,23 |

## ETUDE PHARMACOLOGIQUE DES DERIVES DE L'INVENTION

L'action inhibitrice des composés de la présente invention a été démontrée d'une part au niveau de l'acyl CoA-cholestérol-acyltransférase (ACAT) d'une lignée de macrophages et d'autre part vis-à-vis de la modification oxydative des LDL induite par le sulfate de cuivre in vitro.

### EXEMPLE 58 : Mesure de l'ACAT

L'action des composés de la présente invention au niveau de l'ACAT a été démontrée au moyen d'un test expérimental in vitro utilisant une lignée de macrophages J 774 selon la technique décrite par MAZIERE et col. (Atherosclerosis, 81, 151-160, 1990). Ce test consiste à évaluer l'efficacité d'un produit à inhiber l'estérification du cholestérol libre intracellulaire par l'acide oléique en mesurant la quantité d'oléate de cholestérol radiomarqué formé à la suite de l'incubation d'oléylcoenzyme A radioactif en présence d'un homogénat de macrophages J 774.

Les produits à tester ou le placebo sont incubés en présence des macrophages pendant 24 heures à la concentration de $10^{-5}$M.

L'efficacité des composés est évaluée par le calcul de l'activité de l'ACAT des macrophages incubés en présence d'un produit par rapport à celle des macrophages incubés en présence du placebo et est donc exprimée en pourcentage d'inhibition.

Dans ces conditions, les composés de l'invention ont présenté une activité inhibitrice de l'ACAT des macrophages pouvant aller jusqu'à une inhibition supérieure à 90 %.

C'est le cas, en particulier, des composés des exemples suivants :

exemple 4 : 91 % d'inhibition
exemple 14 : 92 % d'inhibition
exemple 15 : 93 % d'inhibition
exemple 16 : 96 % d'inhibition
exemple 20 : 95 % d'inhibition
exemple 21 : 90 % d'inhibition
exemple 23 : 94 % d'inhibition
exemple 57 : 98 % d'inhibition
exemple 58 : 97 % d'inhibition

Cette activité est d'autant plus intéressante qu'elle est beaucoup plus puissante que celle mesurée pour deux produits de référence : le 2,4,6-triméthoxy-(9-(Z)-octadécènamido)benzène (Ref.1) et le 2,4,6-triméthoxy-(2,2-diméthyldodécanamido)benzène (Ref.2) qui dans les mêmes conditions ne présente que 75 % d'inhibition.

L'$IC_{50}$ du composé décrit dans l'exemple 20 est égale à $2,5.10^{-7}$M, alors que celles des composés de référence sont égales à $3.10^{-6}$M pour Ref.1 et $6.10^{-6}$M pour Ref.2 comme il est indiqué dans la figure suivante :

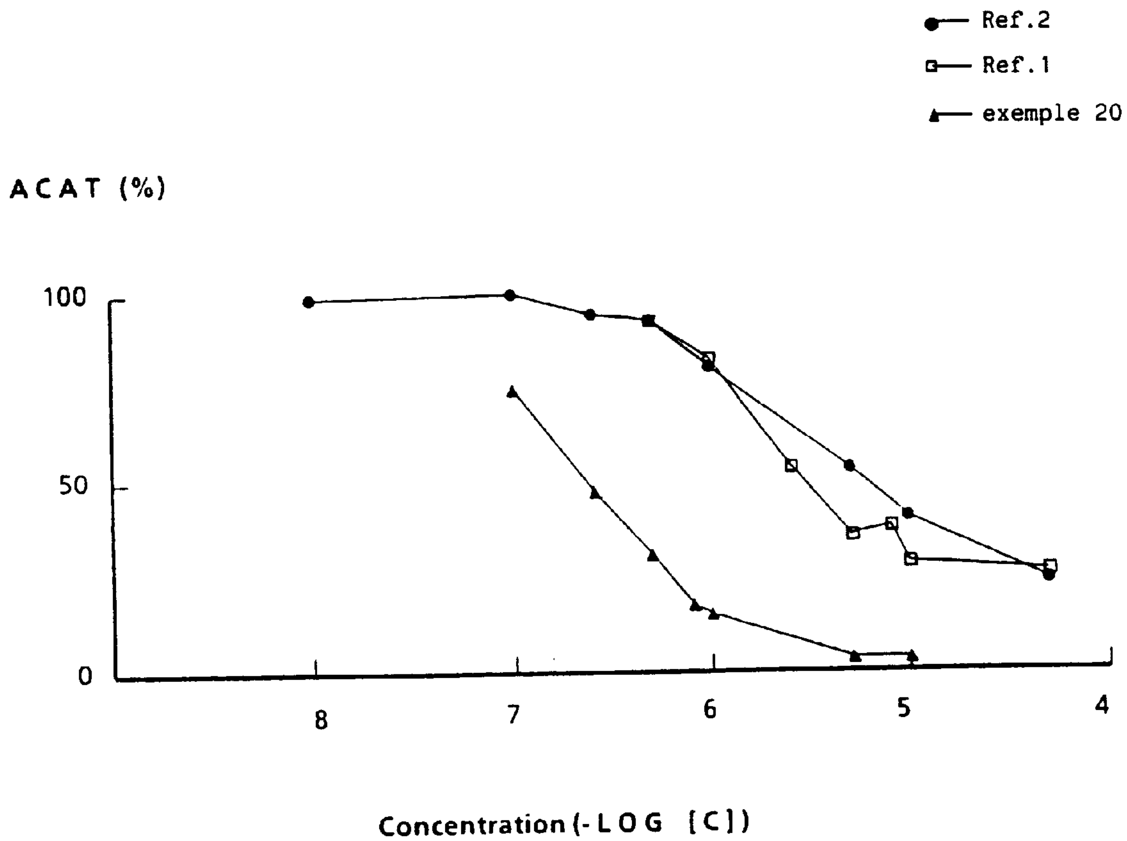


**EXEMPLE 59 : Modification des LDL par le sulfate de cuivre**

Les LDL humaines sont incubées 24 heures en présence de sulfate de cuivre ($5.10^{-6}$M) avec ou sans les composés à tester (concentrations entre $10^{-7}$M et $10^{-4}$M).

Après incubation, la peroxydation des LDL est évaluée par électrophorèse sur gel d'agar et par la formation de l'un des produits de la peroxydation lipidique : le malondialdehyde (MDA) selon la technique décrite par PARTHASARATHY et col. (J. Clin. Invest. 77, 641-644, 1986).

L'activité des composés testés est évaluée par le calcul des concentrations réduisant de 50 % ($IC_{50}$) la production de MDA par rapport au contrôle.

Les composés de l'invention présentent une activité antioxydante vis-à-vis des LDL humaines comparable à celle du probucol pris comme référence. Les $IC_{50}$ se situent entre $3.10^{-6}$ et $5.10^{-6}$ pour les composés de l'invention alors que les produits de référence inhibiteurs d'ACAT (Ref. 1 et Ref. 2) sont complètement dépourvus de cette activité.

Les résultats obtenus pour le composé de l'exemple 20, le probucol et les composés de référence (Ref.1 et Ref.2) sont illustrés dans la figure suivante :

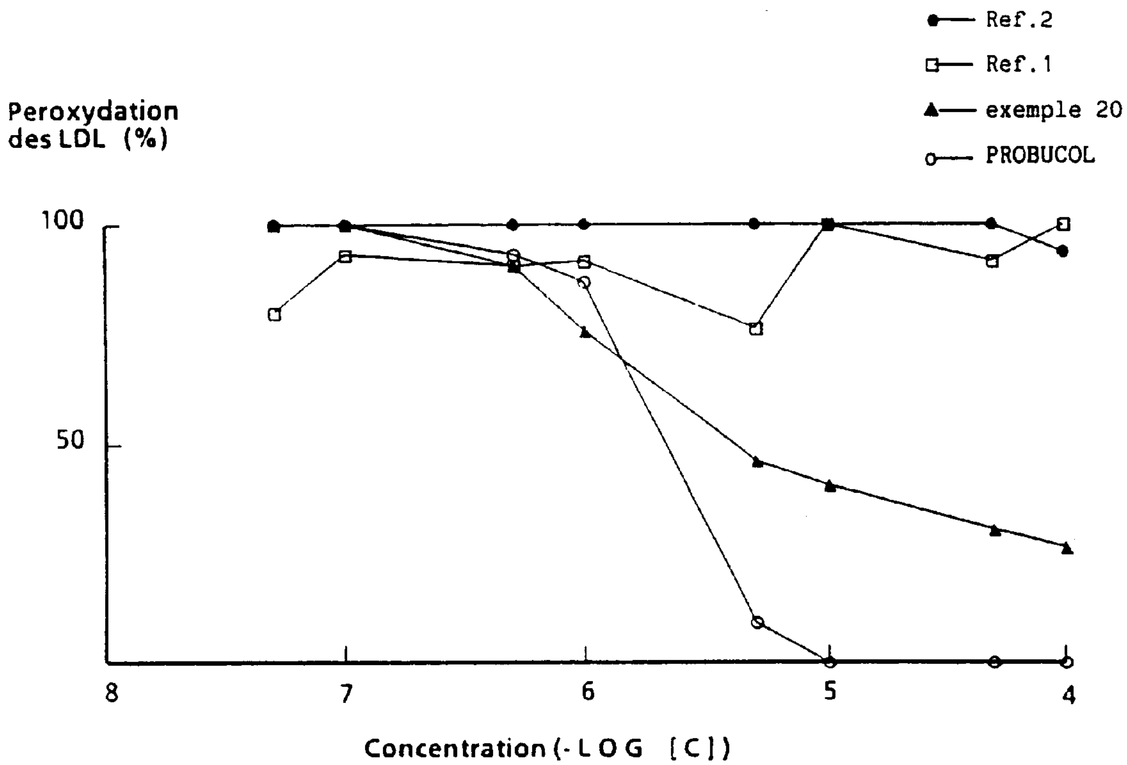


**EXEMPLE 60 : Effet des composés de l'invention chez le hamster hypercholestérolémique**

Des hamsters Golden Syrian sont soumis à un régime hypercholestérolémique de 0,2 % pendant 3 semaines. Ils sont ensuite traités par voie orale soit par le véhicule des composés testés (groupe contrôle), soit par un produit de référence, soit par un produit original à la dose de 10 mg/kg/jour pendant 2 semaines. En fin de traitement, le cholestérol total est mesuré dans le plasma.

Résultats :

Les hamsters soumis au régime enrichi en cholestérol présentent de façon stable une augmentation de 100 % de leur taux de cholestérol plasmatique par rapport aux hamsters soumis à un régime normal. Le composé de l'exemple 20 administré à la dose quotidienne de 10 mg/kg par voie orale, exerce une excellente activité hypocholestérolémiante, normalisant les taux de cholestérol des animaux hypercholestérolémiques au terme des 15 jours de traitement. Cette activité est nettement supérieure à celle des produits de référence testés (Ref.1 et Ref.2) dans les mêmes conditions. Ces résultats sont rassemblés dans la figure suivante :

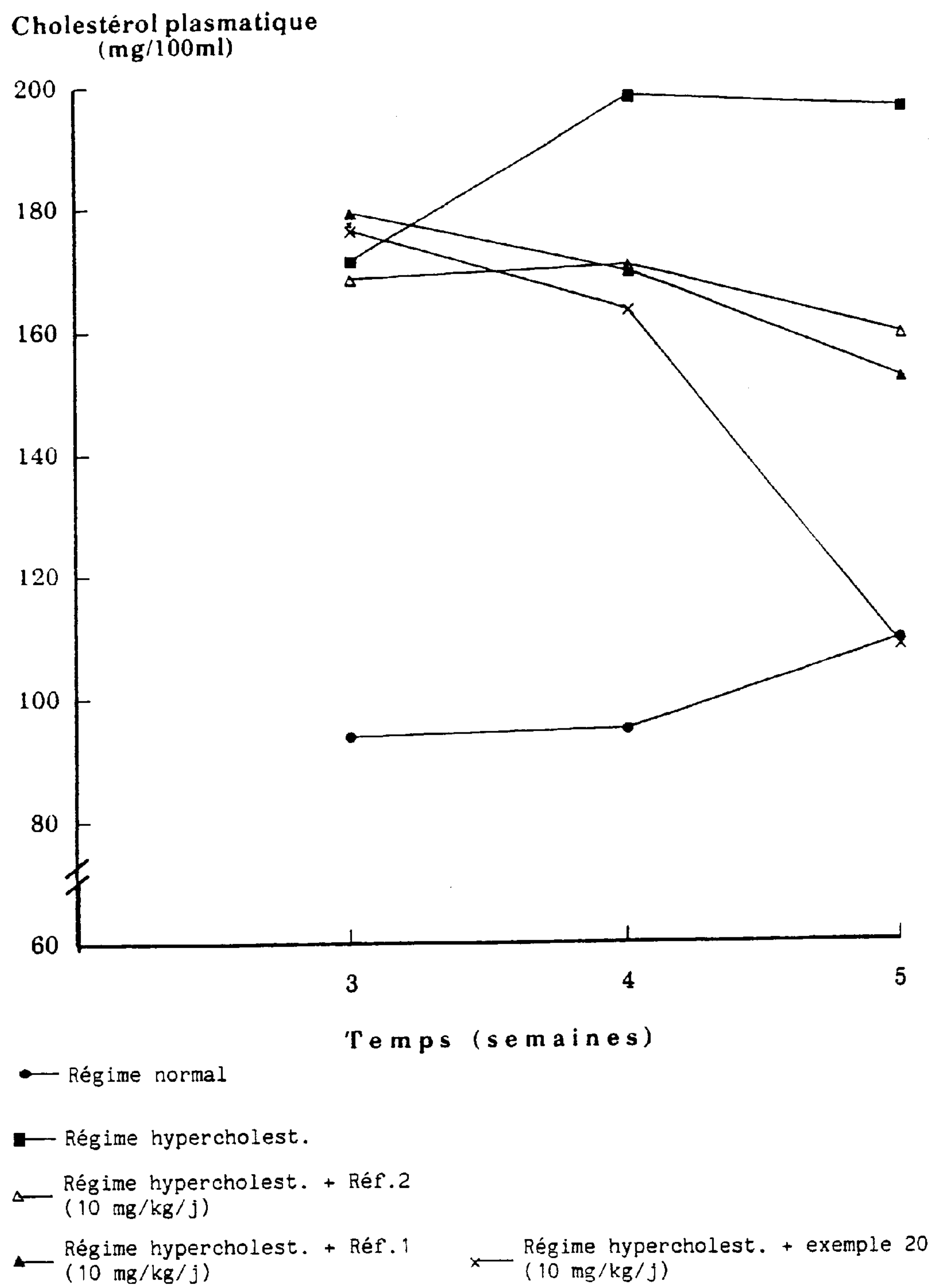


**EXEMPLE 61 : Composition pharmaceutique**

Comprimé : formule de préparation pour 1000 comprimés dosés à 50 mg de principe actif.

| | |
|---|---|
| 2,3,6-Triméthyl-4-(2-méthyl-9-octadécénamido) phénol (isomère cis) | 50 g |
| Hydroxypropylcellulose | 2 g |
| Amidon de blé | 10 g |
| Lactose | 100 g |
| Stéarate de magnésium | 3 g |
| Talc | 3 g |

## Revendications

**Revendications pour les Etats contractants suivants : AT, BE, CH, DE, DK, FR, GB, IT, LI, LU, NL, PT, SE**

**1.** Composés de formule (I) :

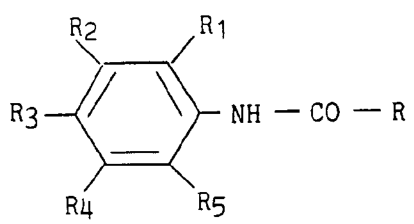

(I)

dans laquelle :

R représente un groupement alkyle ($C_8$-$C_{21}$) linéaire ou ramifié, un groupement 1-[alkyle($C_8$-$C_{21}$)]cycloalk-1-yle($C_3$-$C_6$)linéaire ou ramifié, ou un groupement alcényle ($C_8$-$C_{21}$) linéaire ou ramifié comportant de une à trois double liaisons,

et soit

$R_3$ représente un groupement hydroxy,

$R_1$ et $R_2$, différents, représentent un atome d'hydrogène ou un groupement alkyle ($C_1$-$C_6$) linéaire ou ramifié,

$R_4$ et $R_5$ identiques ou différents représentent un groupement alkyle ($C_1$-$C_6$) linéaire ou ramifié ou un groupement alkoxy ($C_1$-$C_6$) linéaire ou ramifié,

soit

$R_1$ et $R_4$ représentent simultanément un groupement hydroxy,

$R_2$, $R_3$ et $R_5$, identiques ou différents représentent un groupement alkyle ($C_1$-$C_6$) linéaire ou ramifié ou un groupement alkoxy ($C_1$-$C_6$) linéaire ou ramifié,

leurs énantiomères, diastéréoisomères et épimères, leurs isomères cis/trans, ainsi que leurs sels d'addition à une base pharmaceutiquement acceptable.

**2.** Composés selon la revendication 1 tels que $R_3$ représente un groupement hydroxy, leurs énantiomères, diastéréoisomères et épimères, leurs isomères cis/trans ainsi que leurs sels d'addition à une base pharmaceutiquement acceptable.

**3.** Composés selon la revendication 1 tels que $R_1$ et $R_4$ représentent simultanément un groupement hydroxy, leurs énantiomères, diastéréoisomères et épimères, leurs isomères cis/trans ainsi que leurs sels d'addition à une base pharmaceutiquement acceptable.

**4.** Composés selon la revendication 1 tels que $R_3$ représente un groupement hydroxy et $R_2$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**5.** Composés selon la revendication 1 tels que $R_3$ représente un groupement hydroxy et $R_1$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**6.** Composés selon la revendication 1 tels que $R_3$ représente un groupement hydroxy, $R_2$ représente un groupement méthyle, $R_4$ et $R_5$ représentent simultanément un groupement méthoxy, leurs énantiomères, diastéréoisomères et épimères, leurs isomères cis/trans ainsi que leurs sels d'addition à une base pharmaceutiquement acceptable.

**7.** Composés selon la revendication 1 tels que $R_1$ et $R_4$ représentent simultanément un groupement hydroxy et $R_2$, $R_3$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**8.** Composés selon la revendication 1 tels que $R_1$ et $R_4$ représentent simultanément un groupement hydroxy, $R_2$ et $R_3$ représentent simultanément un groupement méthoxy, $R_5$ représente un groupement méthyle, leurs énantiomères, diastéréoisomères et épimères, leurs isomères cis/trans ainsi que leurs sels d'addition à une base pharmaceutiquement acceptable.

**9.** Composé selon l'une quelconque des revendications 1, 2 ou 4 qui est le 2,3,6-triméthyl-4-(2-méthylundécanamido)phénol, ses énantiomères ainsi que ses sels d'addition à une base pharmaceutiquement acceptable.

**10.** Composé selon l'une quelconque des revendications 1, 2 ou 4 qui est le 2,3,6-triméthyl-4-(2,2-diméthylundécanamido)phénol ainsi que ses sels d'addition à une base pharmaceutiquement acceptable.

**11.** Composé selon l'une quelconque des revendications 1, 2 ou 4 qui est le 2,3,6-triméthyl-4-(2-méthyl-9-octadécènamido)phénol, ses énantiomères, isomères cis/trans ainsi que ses sels d'addition à une base pharmaceutiquement acceptable.

**12.** Composé selon l'une quelconque des revendications 1, 2 ou 4 qui est le 2,3,6-triméthyl-4-(9,12-octadécadiénamido)phénol, ses isomères cis/trans ainsi que ses sels d'addition à une base pharmaceutiquement acceptable.

**13.** Composé selon l'une quelconque des revendications 1, 2 ou 4 qui est le 2,3,6-triméthyl-4-(6,9,12-octadécatriènamido)phénol, ses isomères cis/trans ainsi que ses sels d'addition à une base pharmaceutiquement acceptable.

**14.** Procédé de préparation des composés de formule (I) caractérisé en ce que l'on utilise comme matière première un composé de formule (II)

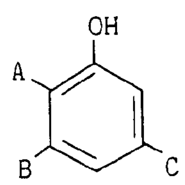

(II)

dans laquelle les substituants A, B ou C identiques ou différents représentent un groupement alkyle ($C_1$-$C_6$) linéaire ou ramifié ou alkoxy ($C_1$-$C_6$) linéaire ou ramifié
que l'on fait réagir :
1
soit en présence de persulfate d'un métal alcalin en présence d'une base,
pour conduire au diphénol de formule (II/a) :

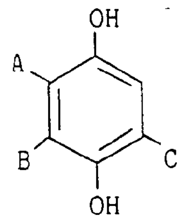

(II/a)

dans laquelle les substituants A, B et C ont la même signification que précédemment,

qui est traité par de l'acide nitrique en milieu acétique, pour conduire à une p.benzoquinone de formule (III) :

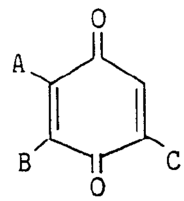

(III)

dans laquelle les substituants A, B et C ont la même signification que précédemment,

qui est ensuite traitée

ou bien

dans le cas où les composés de formule (I) que l'on souhaite obtenir possèdent un groupement $R_3$=OH et dans ce cas A, B et C peuvent être respectivement remplacés par $R_5$, $R_4$ et $R_2$,

en présence d'hydroxylamine en milieu chlorhydrique pour conduire au composé de formule (IV) :

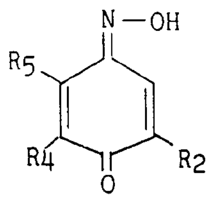

(IV)

dans laquelle :

$R_2$, $R_4$ et $R_5$ ont la même signification que dans la formule (I),

ou bien

dans le cas où les composés de formule (I) que l'on souhaite obtenir possèdent des groupements $R_1$=$R_4$=OH et dans ce cas A, B et C peuvent être respectivement remplacés par $R_2$, $R_3$ et $R_5$,

en présence d'acide chlorhydrique en milieu aqueux puis d'acide nitrique pour conduire au composé de formule (V) :

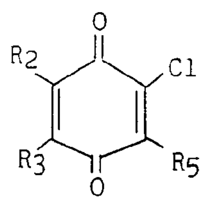

(V)

dans laquelle :

$R_2$, $R_3$ et $R_5$ ont la même signification que dans la formule (I)

que l'on met ensuite en réaction en présence d'azidure de sodium,

pour conduire au composé de formule (VI) :

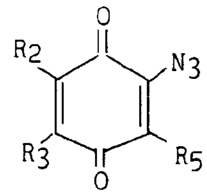

(VI)

dans laquelle $R_2$, $R_3$ et $R_5$ ont la même signification que dans la formule (I)

2

soit en présence de nitrate de sodium et en utilisant le trinitrate de lanthane hexahydraté comme catalyseur en milieu chlorhydrique,

pour conduire à un mélange des composés (VII) et (VIII), que l'on sépare par une technique classique de séparation,

dans le cas où les composés de formule (I) que l'on souhaite obtenir possèdent :

a des groupements $R_1=R_4=OH$ et dans ce cas A, B et C peuvent être respectivement remplacés par $R_2$, $R_3$ et $R_5$

ou

b un groupement $R_3=OH$ et dans ce cas A, B et C peuvent être respectivement remplacés par $R_4$, $R_5$ et $R_1$

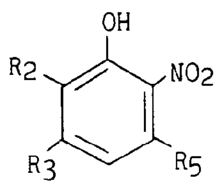

(VII)

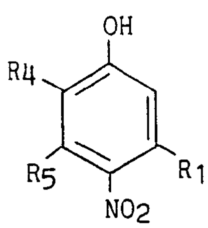

(VIII)

. dans lesquelles :

. $R_1$, $R_2$, $R_3$, $R_4$ et $R_5$ ont la même signification que dans la formule (I),

dérivé de formule (VII) que l'on met en réaction avec une solution aqueuse de persulfate d'un métal alcalin en présence d'une base puis que l'on traite par de l'acide sulfurique concentré,

pour conduire au composé de formule (IX) :

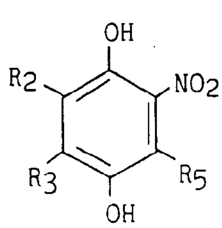

(IX)

dans laquelle :

$R_2$, $R_3$ et $R_5$ ont la même signification que dans la formule (I),

dérivés de formules (IV), (VI), (VIII) et (IX) que l'on soumet à une hydrogénation catalytique,

pour conduire aux composés de formules (X), (XI) et (XII), que l'on conserve sous atmosphère inerte :

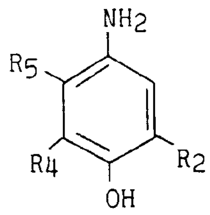

(X)

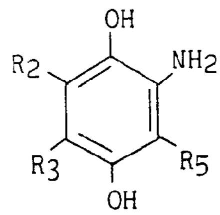

(XI)

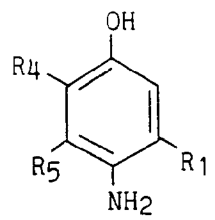

(XII)

dans lesquelles :
$R_1$, $R_2$, $R_3$, $R_4$ et $R_5$ ont la même signification que dans la formule (I),
dérivés de formules (X), (XI) et (XII) que l'on fait réagir, sous atmosphère inerte :
ou bien avec un composé de formule (XIII) :

$$R - CO - O - CO - O - CH_2 - CH_3 \qquad (XIII)$$

dans laquelle R a la même signification que dans la formule (I),
ou bien avec un composé de formule (XIV), en présence d'une base organique :

$$R - CO - Cl \qquad (XIV)$$

dans laquelle R a la même signification que dans la formule (I),
pour conduire respectivement aux composés de formule (I/a), (I/b) et (I/c) qui constituent l'ensemble des composés de formule (I) :

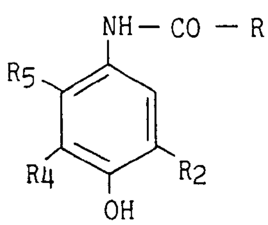

(I/a)

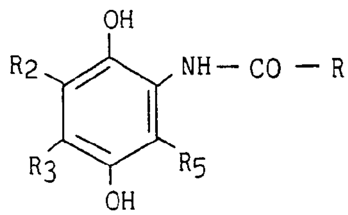

(I/b)

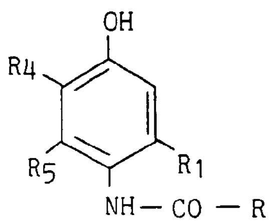

(I/c)

composés de formule (I/a), (I/b), (I/c) que l'on purifie par une technique classique de purification dont on sépare, le cas échéant, les isomères optiques ou cis/trans par des techniques classiques de séparation et que l'on peut transformer en leurs sels d'addition à une base pharmaceutiquement acceptable.

**15.** Compositions pharmaceutiques contenant comme principe actif un composé selon l'une quelconque des revendications 1 à 13, seul ou en combinaison avec un ou plusieurs véhicules inertes, non toxiques pharmaceutiquement acceptable.

**16.** Compositions pharmaceutiques selon la revendication 15 utiles pour le traitement des dyslipidémies et de l'athérosclérose.

**Revendications pour l'Etat contractant suivant : ES**

**1.** Procédé de préparation des composés de formule (I) :

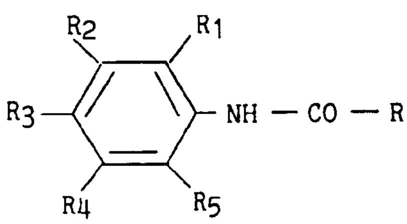

(I)

dans laquelle :

R représente un groupement alkyle ($C_8$-$C_{21}$) linéaire ou ramifié, un groupement 1-[alkyle($C_8$-$C_{21}$)]cycloalk-1-yle($C_3$-$C_6$)linéaire ou ramifié, ou un groupement alcényle ($C_8$-$C_{21}$) linéaire ou ramifié comportant de une à trois double liaisons,

et soit

$R_3$ représente un groupement hydroxy,

$R_1$ et $R_2$, différents, représentent un atome d'hydrogène ou un groupement alkyle ($C_1$-$C_6$) linéaire ou ramifié,

$R_4$ et $R_5$ identiques ou différents représentent un groupement alkyle ($C_1$-$C_6$) linéaire ou ramifié ou un groupement alkoxy ($C_1$-$C_6$) linéaire ou ramifié,

soit

$R_1$ et $R_4$ représentent simultanément un groupement hydroxy,

$R_2$, $R_3$ et $R_5$, identiques ou différents représentent un groupement alkyle ($C_1$-$C_6$) linéaire ou ra-

mifié ou un groupement alkoxy ($C_1$-$C_6$) linéaire ou ramifié,
leurs énantiomères, diastéréoisomères et épimères, leurs isomères cis/trans, ainsi que leurs sels d'addition à une base pharmaceutiquement acceptable,
caractérisé en ce que l'on utilise comme matière première un composé de formule (II)

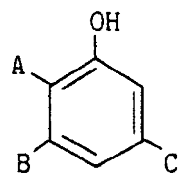

(II)

dans laquelle les substituants A, B ou C identiques ou différents représentent un groupement alkyle ($C_1$-$C_6$) linéaire ou ramifié ou alkoxy ($C_1$-$C_6$) linéaire ou ramifié
que l'on fait réagir :
1
soit en présence de persulfate d'un métal alcalin en présence d'une base,
pour conduire au diphénol de formule (II/a) :

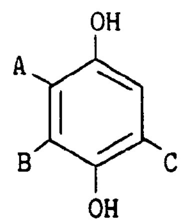

(II/a)

dans laquelle les substituants A, B et C ont la même signification que précédemment,
qui est traité par de l'acide nitrique en milieu acétique, pour conduire à une p.benzoquinone de formule (III) :

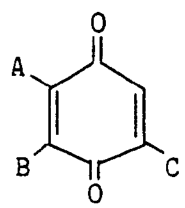

(III)

dans laquelle les substituants A, B et C ont la même signification que précédemment,
qui est ensuite traitée
ou bien
dans le cas où les composés de formule (I) que l'on souhaite obtenir possèdent un groupement $R_3$=OH et dans ce cas A, B et C peuvent être respectivement remplacés par $R_5$, $R_4$ et $R_2$,
en présence d'hydroxylamine en milieu chlorhydrique pour conduire au composé de formule (IV) :

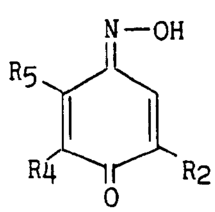

(IV)

dans laquelle :
$R_2$, $R_4$ et $R_5$ ont la même signification que dans la formule (I),
ou bien

dans le cas où les composés de formule (I) que l'on souhaite obtenir possèdent des groupements $R_1$=$R_4$=OH et dans ce cas A, B et C peuvent être respectivement remplacés par $R_2$, $R_3$ et $R_5$,

en présence d'acide chlorhydrique en milieu aqueux puis d'acide nitrique pour conduire au composé de formule (V) :

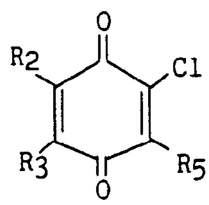

(V)

dans laquelle :

$R_2$, $R_3$ et $R_5$ ont la même signification que dans la formule (I)

que l'on met ensuite en réaction en présence d'azidure de sodium,

pour conduire au composé de formule (VI) :

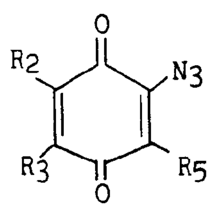

(VI)

dans laquelle $R_2$, $R_3$ et $R_5$ ont la même signification que dans la formule (I)

2

soit en présence de nitrate de sodium et en utilisant le trinitrate de lanthane hexahydraté comme catalyseur en milieu chlorhydrique,

pour conduire à un mélange des composés (VII) et (VIII), que l'on sépare par une technique classique de séparation,

dans le cas où les composés de formule (I) que l'on souhaite obtenir possèdent :

a des groupements $R_1$=$R_4$=OH et dans ce cas A, B et C peuvent être respectivement remplacés par $R_2$, $R_3$ et $R_5$

ou

b un groupement $R_3$=OH et dans ce cas A, B et C peuvent être respectivement remplacés par $R_4$, $R_5$ et $R_1$

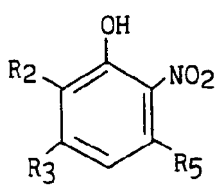

(VII)

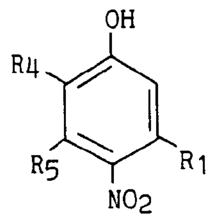

(VIII)

. dans lesquelles :

. $R_1$, $R_2$, $R_3$, $R_4$ et $R_5$ ont la même signification que dans la formule (I),

dérivé de formule (VII) que l'on met en réaction avec une solution aqueuse de persulfate d'un métal alcalin en présence d'une base puis que l'on traite par de l'acide sulfurique concentré,
pour conduire au composé de formule (IX) :

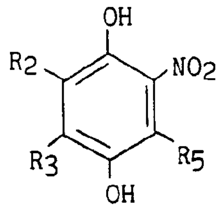

(IX)

dans laquelle :
$R_2$, $R_3$ et $R_5$ ont la même signification que dans la formule (I),
dérivés de formules (IV), (VI), (VIII) et (IX) que l'on soumet à une hydrogénation catalytique,
pour conduire aux composés de formules (X), (XI) et (XII), que l'on conserve sous atmosphère inerte :

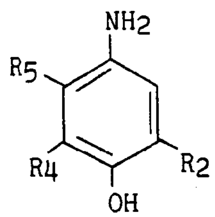

(X)

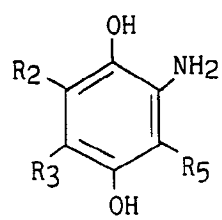

(XI)

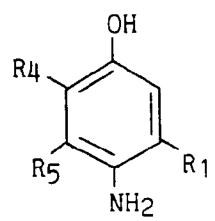

(XII)

dans lesquelles :
$R_1$, $R_2$, $R_3$, $R_4$ et $R_5$ ont la même signification que dans la formule (I),
dérivés de formules (X), (XI) et (XII) que l'on fait réagir, sous atmosphère inerte :
ou bien avec un composé de formule (XIII) :

$$R - CO - O - CO - O - CH_2 - CH_3 \qquad (XIII)$$

dans laquelle R a la même signification que dans la formule (I),
ou bien avec un composé de formule (XIV), en présence d'une base organique :

$$R - CO - Cl \qquad (XIV)$$

dans laquelle R a la même signification que dans la formule (I),
pour conduire respectivement aux composés de formule (I/a), (I/b) et (I/c) qui constituent l'ensemble des composés de formule (I) :

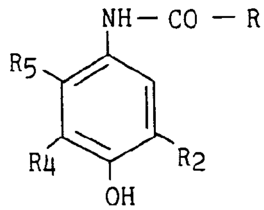

(I/a)

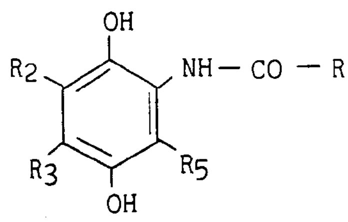

(I/b)

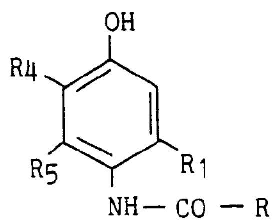

(I/c)

composés de formule (I/a), (I/b), (I/c) que l'on purifie par une technique classique de purification dont on sépare, le cas échéant, les isomères optiques ou cis/trans par des techniques classiques de séparation et que l'on peut transformer en leurs sels d'addition à une base pharmaceutiquement acceptable.

**2.** Procédé de préparation selon la revendication 1 des composés tels que $R_3$ représente un groupement hydroxy, leurs énantiomères, diastéréoisomères et épimères, leurs isomères cis/trans ainsi que leurs sels d'addition à une base pharmaceutiquement acceptable.

**3.** Procédé de préparation selon la revendication 1 des composés tels que $R_1$ et $R_4$ représentent simultanément un groupement hydroxy, leurs énantiomères, diastéréoisomères et épimères, leurs isomères cis/trans ainsi que leurs sels d'addition à une base pharmaceutiquement acceptable.

**4.** Procédé de préparation selon la revendication 1 des composés tels que $R_3$ représente un groupement hydroxy et $R_2$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**5.** Procédé de préparation selon la revendication 1 des composés tels que $R_3$ représente un groupement hydroxy et $R_1$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**6.** Procédé de préparation selon la revendication 1 des composés tels que $R_3$ représente un groupement hydroxy, $R_2$ représente un groupement méthyle, $R_4$ et $R_5$ représentent simultanément un groupement méthoxy, leurs énantiomères, diastéréoisomères et épimères, leurs isomères cis/trans ainsi que leurs sels d'addition à une base pharmaceutiquement acceptable.

**7.** Procédé de préparation selon la revendication 1 des composés tels que $R_1$ et $R_4$ représentent simulta-

nément un groupement hydroxy et $R_2$, $R_3$, $R_5$ représentent simultanément un groupement méthyle, leurs énantiomères, diastéréoisomères et épimères, leurs isomères cis/trans ainsi que leurs sels d'addition à une base pharmaceutiquement acceptable.

8. Procédé de préparation selon la revendication 1 des composés tels que $R_1$ et $R_4$ représentent simultanément un groupement hydroxy, $R_2$ et $R_3$ représentent simultanément un groupement méthoxy, $R_5$ représente un groupement méthyle, leurs énantiomères, diastéréoisomères et épimères, leurs isomères cis-/trans ainsi que leurs sels d'addition à une base pharmaceutiquement acceptable.

9. Procédé de préparation selon l'une quelconque des revendications 1, 2 ou 4 du composé qui est le 2,3,6-triméthyl-4-(2-méthylundécanamido)phénol, ses énantiomères ainsi que ses sels d'addition à une base pharmaceutiquement acceptable.

10. Procédé de préparation selon l'une quelconque des revendications 1, 2 ou 4 du composé qui est le 2,3,6-triméthyl-4-(2,2-diméthylundécanamido)phénol ainsi que ses sels d'addition à une base pharmaceutiquement acceptable.

11. Procédé de préparation selon l'une quelconque des revendications 1, 2 ou 4 du composé qui est le 2,3,6-triméthyl-4-(2-méthyl-9-octadécènamido)phénol, ses énantiomères, isomères cis/trans ainsi que ses sels d'addition à une base pharmaceutiquement acceptable.

12. Procédé de préparation selon l'une quelconque des revendications 1, 2 ou 4 du composé qui est le 2,3,6-triméthyl-4-(9,12-octadécadiénamido)phénol, ses isomères cis/trans ainsi que ses sels d'addition à une base pharmaceutiquement acceptable.

13. Procédé de préparation selon l'une quelconque des revendications 1, 2 ou 4 du composé qui est le 2,3,6-triméthyl-4-(6,9,12-octadécatriènamido)phénol, ses isomères cis/trans ainsi que ses sels d'addition à une base pharmaceutiquement acceptable.

**Revendications pour l'Etat contractant suivant : GR**

1. Procédé de préparation des composés de formule (I) :

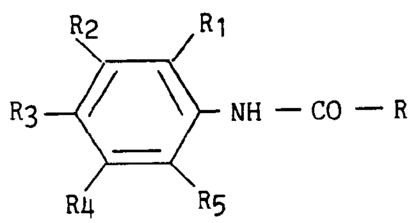

(I)

dans laquelle :

R représente un groupement alkyle ($C_8$-$C_{21}$) linéaire ou ramifié, un groupement 1-[alkyle($C_8$-$C_{21}$)]cycloalk-1-yle($C_3$-$C_6$)linéaire ou ramifié, ou un groupement alcényle ($C_8$-$C_{21}$) linéaire ou ramifié comportant de une à trois double liaisons,

et soit

$R_3$ représente un groupement hydroxy,

$R_1$ et $R_2$, différents, représentent un atome d'hydrogène ou un groupement alkyle ($C_1$-$C_6$) linéaire ou ramifié,

$R_4$ et $R_5$ identiques ou différents représentent un groupement alkyle ($C_1$-$C_6$) linéaire ou ramifié ou un groupement alkoxy ($C_1$-$C_6$) linéaire ou ramifié,

soit

$R_1$ et $R_4$ représentent simultanément un groupement hydroxy,

$R_2$, $R_3$ et $R_5$, identiques ou différents représentent un groupement alkyle ($C_1$-$C_6$) linéaire ou ramifié ou un groupement alkoxy ($C_1$-$C_6$) linéaire ou ramifié,

leurs énantiomères, diastéréoisomères et épimères, leurs isomères cis/trans, ainsi que leurs sels d'addition à une base pharmaceutiquement acceptable,

caractérisé en ce que l'on utilise comme matière première un composé de formule (II)

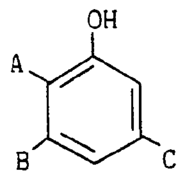

(II)

dans laquelle les substituants A, B ou C identiques ou différents représentent un groupement alkyle ($C_1$-$C_6$) linéaire ou ramifié ou alkoxy ($C_1$-$C_6$) linéaire ou ramifié
que l'on fait réagir :
1
soit en présence de persulfate d'un métal alcalin en présence d'une base,
pour conduire au diphénol de formule (II/a) :

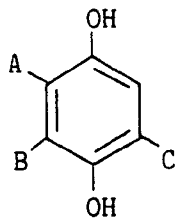

(II/a)

dans laquelle les substituants A, B et C ont la même signification que précédemment,
qui est traité par de l'acide nitrique en milieu acétique, pour conduire à une p.benzoquinone de formule (III) :

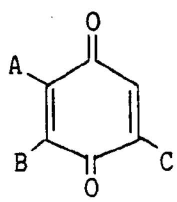

(III)

dans laquelle les substituants A, B et C ont la même signification que précédemment,
qui est ensuite traitée
ou bien
dans le cas où les composés de formule (I) que l'on souhaite obtenir possèdent un groupement $R_3$=OH et dans ce cas A, B et C peuvent être respectivement remplacés par $R_5$, $R_4$ et $R_2$,
en présence d'hydroxylamine en milieu chlorhydrique pour conduire au composé de formule (IV) :

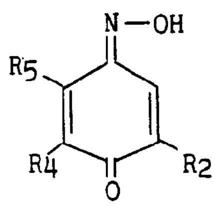

(IV)

dans laquelle :
$R_2$, $R_4$ et $R_5$ ont la même signification que dans la formule (I),
ou bien
dans le cas où les composés de formule (I) que l'on souhaite obtenir possèdent des groupements $R_1$=$R_4$=OH et dans ce cas A, B et C peuvent être respectivement remplacés par $R_2$, $R_3$ et $R_5$,
en présence d'acide chlorhydrique en milieu aqueux puis d'acide nitrique pour conduire au composé de formule (V) :

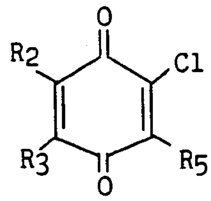

(V)

dans laquelle :
$R_2$, $R_3$ et $R_5$ ont la même signification que dans la formule (I)
que l'on met ensuite en réaction en présence d'azidure de sodium,
pour conduire au composé de formule (VI) :

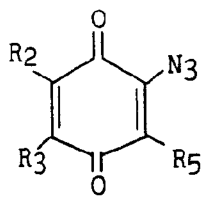

(VI)

dans laquelle $R_2$, $R_3$ et $R_5$ ont la même signification que dans la formule (I)

2
soit en présence de nitrate de sodium et en utilisant le trinitrate de lanthane hexahydraté comme catalyseur en milieu chlorhydrique,

pour conduire à un mélange des composés (VII) et (VIII), que l'on sépare par une technique classique de séparation,

dans le cas où les composés de formule (I) que l'on souhaite obtenir possèdent :

a des groupements $R_1$=$R_4$=OH et dans ce cas A, B et C peuvent être respectivement remplacés par $R_2$, $R_3$ et $R_5$

ou

b un groupement $R_3$=OH et dans ce cas A, B et C peuvent être respectivement remplacés par $R_4$, $R_5$ et $R_1$

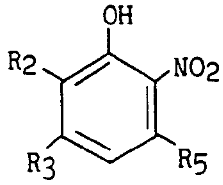

(VII)

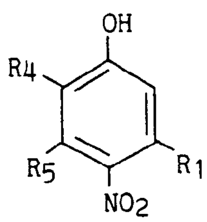

(VIII)

. dans lesquelles :

. $R_1$, $R_2$, $R_3$, $R_4$ et $R_5$ ont la même signification que dans la formule (I),

dérivé de formule (VII) que l'on met en réaction avec une solution aqueuse de persulfate d'un métal alcalin en présence d'une base puis que l'on traite par de l'acide sulfurique concentré,
pour conduire au composé de formule (IX) :

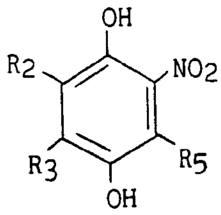

(IX)

dans laquelle :
$R_2$, $R_3$ et $R_5$ ont la même signification que dans la formule (I),
dérivés de formules (IV), (VI), (VIII) et (IX) que l'on soumet à une hydrogénation catalytique,
pour conduire aux composés de formules (X), (XI) et (XII), que l'on conserve sous atmosphère inerte :

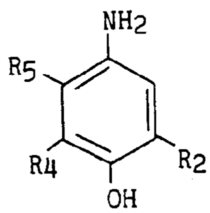

(X)

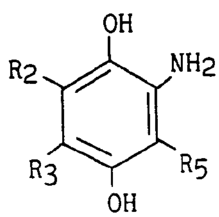

(XI)

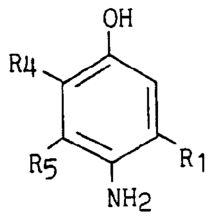

(XII)

dans lesquelles :
$R_1$, $R_2$, $R_3$, $R_4$ et $R_5$ ont la même signification que dans la formule (I),
dérivés de formules (X), (XI) et (XII) que l'on fait réagir, sous atmosphère inerte :
ou bien avec un composé de formule (XIII) :

$$R - CO - O - CO - O - CH_2 - CH_3 \qquad (XIII)$$

dans laquelle R a la même signification que dans la formule (I),
ou bien avec un composé de formule (XIV), en présence d'une base organique :

$$R - CO - Cl \qquad (XIV)$$

dans laquelle R a la même signification que dans la formule (I),
pour conduire respectivement aux composés de formule (I/a), (I/b) et (I/c) qui constituent l'ensemble des composés de formule (I) :

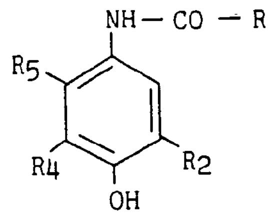

(I/a)

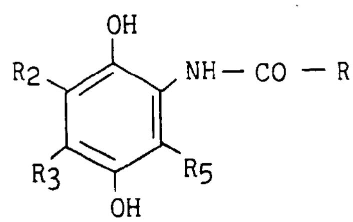

(I/b)

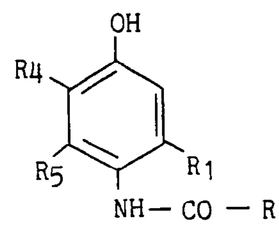

(I/c)

composés de formule (I/a), (I/b), (I/c) que l'on purifie par une technique classique de purification dont on sépare, le cas échéant, les isomères optiques ou cis/trans par des techniques classiques de séparation et que l'on peut transformer en leurs sels d'addition à une base pharmaceutiquement acceptable.

2. Procédé de préparation selon la revendication 1 des composés tels que $R_3$ représente un groupement hydroxy, leurs énantiomères, diastéréoisomères et épimères, leurs isomères cis/trans ainsi que leurs sels d'addition à une base pharmaceutiquement acceptable.

3. Procédé de préparation selon la revendication 1 des composés tels que $R_1$ et $R_4$ représentent simultanément un groupement hydroxy, leurs énantiomères, diastéréoisomères et épimères, leurs isomères cis/trans ainsi que leurs sels d'addition à une base pharmaceutiquement acceptable.

4. Procédé de préparation selon la revendication 1 des composés tels que $R_3$ représente un groupement hydroxy et $R_2$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

5. Procédé de préparation selon la revendication 1 des composes tels que $R_3$ représente un groupement hydroxy et $R_1$, $R_4$, $R_5$ représentent simultanément un groupement méthyle, leurs énantiomères, diastéréoisomères et épimères, leurs isomères cis/trans ainsi que leurs sels d'addition à une base pharmaceutiquement acceptable.

6. Procédé de préparation selon la revendication 1 des composés tels que $R_3$ représente un groupement hydroxy, $R_2$ représente un groupement méthyle, $R_4$ et $R_5$ représentent simultanément un groupement méthoxy, leurs énantiomères, diastéréoisomères et épimères, leurs isomères cis/trans ainsi que leurs sels d'addition à une base pharmaceutiquement acceptable.

7. Procédé de préparation selon la revendication 1 des composés tels que $R_1$ et $R_4$ représentent simulta-

nément un groupement hydroxy et $R_2$, $R_3$, $R_5$ représentent simultanément un groupement méthyle, leurs énantiomères, diastéréoisomères et épimères, leurs isomères cis/trans ainsi que leurs sels d'addition à une base pharmaceutiquement acceptable.

**8.** Procédé de préparation selon la revendication 1 des composés tels que $R_1$ et $R_4$ représentent simultanément un groupement hydroxy, $R_2$ et $R_3$ représentent simultanément un groupement méthoxy, $R_5$ représente un groupement méthyle, leurs énantiomères, diastéréoisomères et épimères, leurs isomères cis/trans ainsi que leurs sels d'addition à une base pharmaceutiquement acceptable.

**9.** Procédé de préparation selon l'une quelconque des revendications 1, 2 ou 4 du composé qui est le 2,3,6-triméthyl-4-(2-méthylundécanamido)phénol, ses énantiomères ainsi que ses sels d'addition à une base pharmaceutiquement acceptable.

**10.** Procédé de préparation selon l'une quelconque des revendications 1, 2 ou 4 du composé qui est le 2,3,6-triméthyl-4-(2,2-diméthylundécanamido)phénol ainsi que ses sels d'addition à une base pharmaceutiquement acceptable.

**11.** Procédé de préparation selon l'une quelconque des revendications 1, 2 ou 4 du composé qui est le 2,3,6-triméthyl-4-(2-méthyl-9-octadécènamido)phénol, ses énantiomères, isomères cis/trans ainsi que ses sels d'addition à une base pharmaceutiquement acceptable.

**12.** Procédé de préparation selon l'une quelconque des revendications 1, 2 ou 4 du composé qui est le 2,3,6-triméthyl-4-(9,12-octadécadiénamido)phénol, ses isomères cis/trans ainsi que ses sels d'addition à une base pharmaceutiquement acceptable.

**13.** Procédé de préparation selon l'une quelconque des revendications 1, 2 ou 4 du composé qui est le 2,3,6-triméthyl-4-(6,9,12-octadécatriènamido)phénol, ses isomères cis/trans ainsi que ses sels d'addition à une base pharmaceutiquement acceptable.

## Patentansprüche

### Patentansprüche für folgende Vertragsstaaten : AT, BE, CH, DE, DK, FR, GB, IT, LI, LU, NL, PT, SE

**1.** Verbindungen der Formel (I):

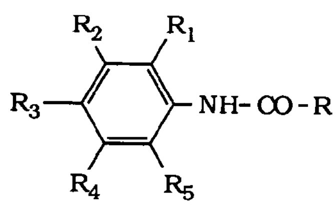

(I)

in der

R eine geradkettige oder verzweigte ($C_8C_{21}$)-Alkylgruppe, eine geradkettige oder verzweigte 1-[($C_8$-$C_{21}$)-Alkyl]-1-($C_3$-$C_6$)-Cycloalkylgruppe oder eine geradkettige oder verzweigte ($C_8$-$C_{21}$)-Alkenylgruppe, die eine bis drei Doppelbindungen aufweist,

und <u>entweder</u>

$R_3$ eine Hydroxylgruppe,

$R_1$ und $R_2$, die voneinander verschieden sind, Wasserstoffatome oder geradkettige oder verzweigte ($C_1$-$C_6$)-Alkylgruppen,

$R_4$ und $R_5$, die gleichartig oder verschieden sein können, eine geradkettige oder verzweigte ($C_1$-$C_6$)-Alkylgruppe oder geradkettige oder verzweigte ($C_1$-$C_6$)-Alkoxygruppe,

<u>oder</u>

$R_1$ und $R_4$ gleichzeitig eine Hydroxylgruppe und

$R_2$, $R_3$ und $R_5$, die gleichartig oder verschieden sein können, geradkettige oder verzweigte ($C_1$-$C_6$)-Alkylgruppen oder geradkettige oder verzweigte ($C_1$-$C_6$)-Alkoxygruppen bedeuten,

deren Enantiomere, Diastereoisomere und Epimere, deren cis/trans-Isomere sowie deren Additionssalze mit einer pharmazeutisch annehmbaren Base.

2. Verbindungen nach Anspruch 1, worin $R_3$ eine Hydroxylgruppe darstellt, deren Enantiomere, Diastereoisomere und Epimere, deren cis/trans-Isomere sowie deren Additionssalze mit einer pharmazeutisch annehmbaren Base.

3. Verbindungen nach Anspruch 1, worin $R_1$ und $R_4$ gleichzeitig eine Hydroxylgruppe bedeuten, deren Enantiomere, Diastereoisomere und Epimere, deren cis/trans-Isomere sowie deren Additionssalze mit einer pharmazeutisch annehmbaren Base.

4. Verbindungen nach Anspruch 1, worin $R_3$ eine Hydroxylgruppe und $R_2$, $R_4$ und $R_5$ gleichzeitig eine Methylgruppe bedeuten, deren Enantiomere, Diastereoisomere und Epimere, deren cis/trans-Isomere und deren Additionssalze mit einer pharmazeutisch annehmbaren Base.

5. Verbindungen nach Anspruch 1, worin R3 eine Hydroxylgruppe und $R_1$, $R_4$ und $R_5$ gleichzeitig eine Methylgruppe bedeuten, deren Enantiomere, Diastereoisomere und Epimere, deren cis/trans-Isomere und deren Additionssalze mit eine pharmazeutisch annehmbaren Base.

6. Verbindungen nach Anspruch 1, worin $R_3$ eine Hydroxylgruppe, $R_2$ eine Methylgruppe und $R_4$ und $R_5$ gleichzeitig eine Methoxygruppe bedeuten, deren Enantiomere, Diastereoisomere und Epimere, deren cis/trans-Isomere sowie deren Additionssalze mit einer pharmazeutisch annehmbaren Base.

7. Verbindungen nach Anspruch 1, worin $R_1$ und $R_4$ gleichzeitig eine Hydroxylgruppe und $R_2$, $R_3$ und $R_5$ gleichzeitig eine Methylgruppe bedeuten, deren Enantiomere, Diastereoisomere und Epimere, deren cis/trans-Isomere sowie deren Additionssalze mit einer pharmazeutisch annehmbaren Base.

8. Verbindungen nach Anspruch 1, worin $R_1$ und $R_4$ gleichzeitig eine Hydroxylgruppe, $R_2$ und $R_3$ gleichzeitig eine Methoxygruppe und $R_5$ eine Methylgruppe bedeuten, deren Enantiomere, Diastereoisomere und Epimere, deren cis/trans-Isomere sowie deren Additionssalze mit einer pharmazeutisch annehmbaren Base.

9. Verbindung nach einem der Ansprüche 1, 2 oder 4, nämlich 2,3,6-Trimethyl-4-(2-methylundecanamido)-phenol, dessen Enantiomere sowie dessen Additionssalze mit einer pharmazeutisch annehmbaren Base.

10. Verbindung nach einem der Ansprüche 1, 2 oder 4, nämlich 2,3,6-Trimethyl-4-(2,2-dimethylundecanamido)-phenol sowie dessen Additionssalze mit einer pharmazeutisch annehmbaren Base.

11. Verbindung nach einem der Ansprüche 1, 2 oder 4, nämlich 2,3,6-Trimethyl-4-(2-methyl-9-octadecenamido)-phenol, dessen Enantiomere, cis/trans-Isomere sowie deren Additionssalze mit einer pharmazeutisch annehmbaren Base.

12. Verbindung nach einem der Ansprüche 1, 2 oder 4, nämlich 2,3,6-Trimethyl-4-(9,12-octadecadienamido)-phenol, dessen cis/trans-Isomere sowie dessen Additionssalze mit einer pharmazeutisch annehmbaren Base.

13. Verbindung nach einem der Ansprüche 1, 2 oder 4, nämlich 2,3,6-Trimethyl-4-(6,9,12-octadecatrienamido)-phenol, dessen cis/trans-Isomere sowie dessen Additionssalze mit einer pharmazeutisch annehmbaren Base.

14. Verfahren zur Herstellung der Verbindungen der Formel (I), **dadurch gekennzeichnet,** daß man als Ausgangsmaterial eine Verbindung der Formel (II)

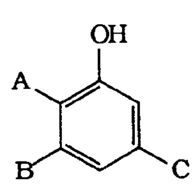

(II)

verwendet, in der die Substituenten A, B oder C, die gleichartig oder verschieden sein können, eine geradkettige oder verzweigte ($C_1$-$C_6$)-Alkylgruppe oder geradkettige oder verzweigte ($C_1$-$C_6$)-Alkoxygruppe bedeuten,
welche man
1
entweder
in Gegenwart einer Base mit einem Alkalimetallpersulfat zu einem Diphenol der Formel (II/a) umsetzt:

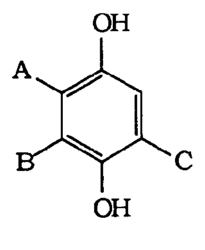

(II/a)

in der die Substituenten A, B und C die oben angegebenen Bedeutungen besitzen,
welches man mit Salpetersäure in essigsaurem Medium behandelt zur Bildung eines p-Benzochinons der Formel (III):

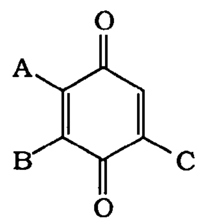

(III)

in der die Substituenten A, B und C die oben angegebenen Bedeutungen besitzen,
welches anschließend
entweder
wenn die Verbindungen der Formel (I), die man herzustellen wünscht, eine Gruppe $R_3$=OH aufweisen und in diesem Fall A, B und C durch $R_5$, $R_4$ bzw. $R_2$ ersetzt sein können,
in chlorwasserstoffsaurem Medium mit Hydroxylamin behandelt wird zur Bildung einer Verbindung der Formel (IV):

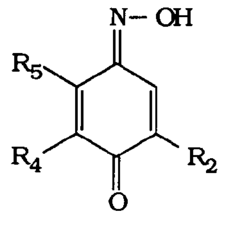

(IV)

in der
$R_2$, $R_4$ und $R_5$ die bezüglich der Formel (I) angegebenen Bedeutungen besitzen,
oder
wenn die Verbindungen der Formel (I), die man herzustellen wünscht, die Gruppen $R_1$=$R_4$=OH aufweisen und in diesem Fall A, B und C durch $R_2$, $R_3$ bzw. $R_5$ ersetzt sein können,
in wäßrigem Medium und in Gegenwart von Chlorwasserstoffsäure mit Salpetersäure behandelt zur Bildung der Verbindung der Formel (V):

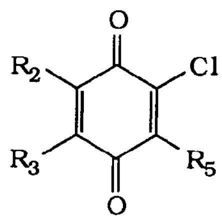

(V)

in der
$R_2$, $R_3$ und $R_5$ die bezüglich der Formel (I) angegebenen Bedeutungen besitzen,
welche man anschließend mit Natriumazid umsetzt zur Bildung der Verbindung der Formel (VI):

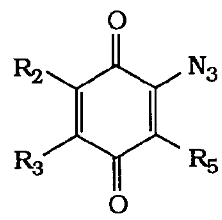

(VI)

in der $R_2$, $R_3$ und $R_5$ die bezüglich der Formel (I) angegebenen Bedeutungen besitzen,

**2**

oder

in chlorwasserstoffsaurem Medium und unter Verwendung von Lanthantrinitrat-hexahydrat als Katalysator mit Natriumnitrat umsetzt

zur Bildung einer Mischung der Verbindungen (VII) und (VIII), die man mit Hilfe einer klassischen Trennmethode trennt, für den Fall, wo die Verbindungen der Formel (I), die man herzustellen wünscht,

a die Gruppen $R_1=R_4=OH$ besitzen und in diesem Fall A, B und C durch $R_2$, $R_3$ bzw. $R_5$ ersetzt sein können, oder

b eine Gruppe $R_3=OH$ und in diesem Fall A, B und C durch $R_4$, $R_5$ bzw. $R_1$ ersetzt sein können,

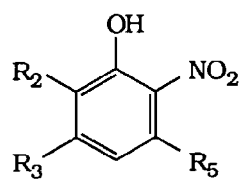

(VII)

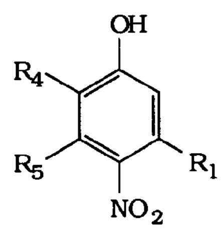

(VIII)

. worin:

. $R_1$, $R_2$, $R_3$, $R_4$ und $R_5$ die bezüglich der Formel (I) angegebenen Bedeutungen besitzen,

welches Derivat der Formel (VII) man in Gegenwart einer Base mit einer wäßrigen Lösung eines Alkalimetallpersulfats umsetzt und dann mit konzentrierter Schwefelsäure behandelt zur Bildung der Verbindung der Formel (IX):

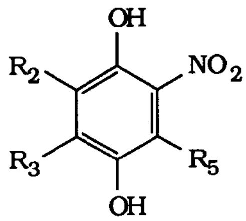

(IX)

in der:

$R_2$, $R_3$ und $R_5$ die bezüglich der Formel (I) angegebenen Bedeutungen besitzen, welche Derivate der Formel (IV), (VI), (VIII) und (IX) man einer katalytischen Hydrierung unterwirft,

zur Bildung der Verbindungen der Formel (X), (XI) und (XII), welche man unter einer inerten Atmosphäre aufbewahrt:

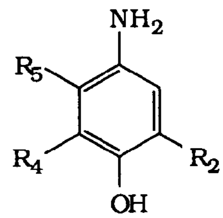

(X)

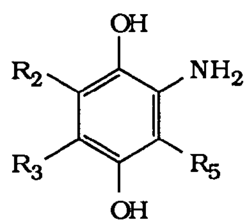

(XI)

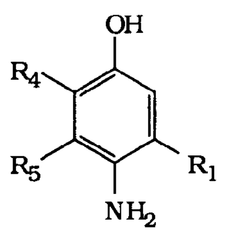

(XII)

worin

$R_1$, $R_2$, $R_3$, $R_4$ und $R_5$ die bezüglich der Formel (I) angegebenen Bedeutungen besitzen,

welche Derivate der Formel (X), (XI) und (XII) man unter einer inerten Atmosphäre <u>entweder</u> mit einer Verbindung der Formel (XIII):

$$R—CO\text{-}O—CO\text{-}O—CH_2—CH_3 \qquad (XIII)$$

in der R die bezüglich der Formel (I) angegebenen Bedeutungen besitzt,

<u>oder</u> mit einer Verbindung der Formel (XIV) in Gegenwart einer organischen Base:

$$R—CO\text{-}Cl \qquad (XIV)$$

in der R die bezüglich der Formel (I) angegebenen Bedeutungen besitzt, umsetzt, so daß man Verbindungen der Formeln (I/a), (I/b) bzw. (I/c) erhält, welche gemeinsam die Verbindungen der Formel (I) darstellen:

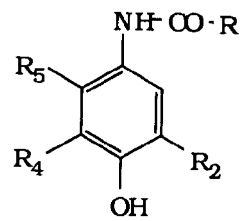

(I/a)

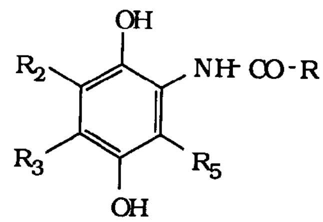

(I/b)

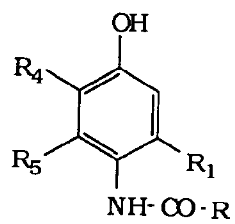

(I/c)

welche Verbindungen der Formeln (I/a), (I/b) und (I/c) man mit Hilfe einer klassischen Reinigungsmethode reinigt und gegebenenfalls mit Hilfe klassischer Trennungsmethoden in die optischen Isomeren oder die cis/trans-Isomeren auftrennt und die man mit einer pharmazeutisch annehmbaren Base in ihre Additionssalze umwandeln kann.

**15.** Pharmazeutische Zubereitungen enthaltend als Wirkstoff eine Verbindung nach einem der Ansprüche 1 bis 13 allein oder in Kombination mit einem oder mehreren inerten, nichttoxischen, pharmazeutisch annehmbaren Trägermateriallen.

**16.** Pharmazeutische Zubereitungen nach Anspruch 15 zur Behandlung von Dyslipidämien und Arteriosklerose.

**Patentansprüche für folgenden Vertragsstaat : ES**

**1.** Verfahren zur Herstellung der Verbindungen der Formel (I):

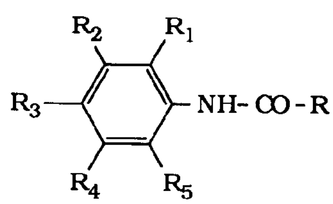

(I)

in der

R eine geradkettige oder verzweigte ($C_8C_{21}$)-Alkylgruppe, eine geradkettige oder verzweigte 1-[($C_8$-$C_{21}$)-Alkyl]-1-($C_3$-$C_6$)-Cycloalkylgruppe oder eine geradkettige oder verzweigte ($C_8$-$C_{21}$)-Alkenylgruppe, die eine bis drei Doppelbindungen aufweist,

und <u>entweder</u>

$R_3$ eine Hydroxylgruppe,

$R_1$ und $R_2$, die voneinander verschieden sind, Wasserstoffatome oder geradkettige oder verzweigte ($C_1$-$C_6$)-Alkylgruppen,

$R_4$ und $R_5$, die gleichartig oder verschieden sein können, eine geradkettige oder verzweigte ($C_1$-$C_6$)-Alkylgruppe oder geradkettige oder verzweigte ($C_1$-$C_6$)-Alkoxygruppe,

oder

$R_1$ und $R_4$ gleichzeitig eine Hydroxylgruppe und

$R_2$, $R_3$ und $R_5$, die gleichartig oder verschieden sein können, geradkettige oder verzweigte ($C_1$-$C_6$)-Alkylgruppen oder geradkettige oder verzweigte ($C_1$-$C_6$)-Alkoxygruppen bedeuten,

von deren Enantiomeren, Diastereoisomeren und Epimeren, deren cis/trans-Isomeren sowie deren Additionssalzen mit einer pharmazeutisch annehmbaren Base,

**dadurch gekennzeichnet,** daß man als Ausgangsmaterial eine Verbindung der Formel (II)

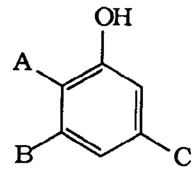

(II)

verwendet, in der die Substituenten A, B oder C, die gleichartig oder verschieden sein können, eine geradkettige oder verzweigte ($C_1$-$C_6$)-Alkylgruppe oder geradkettige oder verzweigte ($C_1$-$C_6$)-Alkoxygruppe bedeuten, welche man

**1**

entweder

in Gegenwart einer Base mit einem Alkalimetallpersulfat zu einem Diphenol der Formel (II/a) umsetzt:

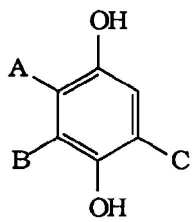

(II/a)

in der die Substituenten A, B und C die oben angegebenen Bedeutungen besitzen,

welches man mit Salpetersäure in essigsaurem Medium behandelt zur Bildung eines p-Benzochinons der Formel (III):

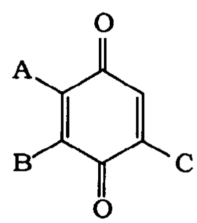

(III)

in der die Substituenten A, B und C die oben angegebenen Bedeutungen besitzen,

welches anschließend

entweder

wenn die Verbindungen der Formel (I), die man herzustellen wünscht, eine Gruppe $R_3$=OH aufweisen und in diesem Fall A, B und C durch $R_5$, $R_4$ bzw. $R_2$ ersetzt sein können,

in chlorwasserstoffsaurem Medium mit Hydroxylamin behandelt wird zur Bildung einer Verbindung der Formel (IV):

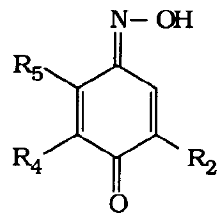

(IV)

in der

$R_2$, $R_4$ und $R_5$ die bezüglich der Formel (I) angegebenen Bedeutungen besitzen,

oder

wenn die Verbindungen der Formel (I), die man herzustellen wünscht, die Gruppen $R_1$=$R_4$=OH aufweisen und in diesem Fall A, B und C durch $R_2$, $R_3$ bzw. $R_5$ ersetzt sein können,

in wäßrigem Medium und in Gegenwart von Chlorwasserstoffsäure mit Salpetersäure behandelt zur Bildung der Verbindung der Formel (V):

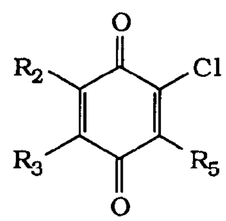

(V)

in der

$R_2$, $R_3$ und $R_5$ die bezüglich der Formel (I) angegebenen Bedeutungen besitzen,

welche man anschließend mit Natriumazid umsetzt zur Bildung der Verbindung der Formel (VI):

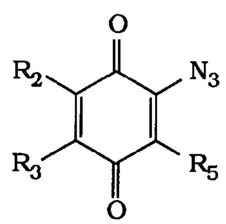

(VI)

in der $R_2$, $R_3$ und $R_5$ die bezüglich der Formel (I) angegebenen Bedeutungen besitzen,

**2**

oder

in chlorwasserstoffsaurem Medium und unter Verwendung von Lanthantrinitrat-hexahydrat als Katalysator mit Natriumnitrat umsetzt

zur Bildung einer Mischung der Verbindungen (VII) und (VIII), die man mit Hilfe einer klassischen Trennmethode trennt, für den Fall, wo die Verbindungen der Formel (I), die man herzustellen wünscht,

a die Gruppen $R_1$=$R_4$=OH besitzen und in diesem FallA, B und C durch $R_2$, $R_3$ bzw. $R_5$ ersetzt sein können, oder

b eine Gruppe $R_3$=OH und in diesem Fall A, B und C durch $R_4$, $R_5$ bzw. $R_1$ ersetzt sein können,

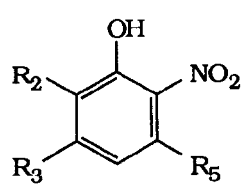

(VII)

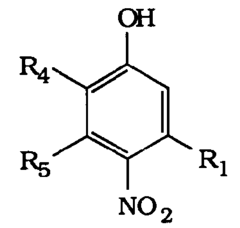

(VIII)

. worin:

. $R_1$, $R_2$, $R_3$, $R_4$ und $R_5$ die bezüglich der Formel (I) angegebenen Bedeutungen besitzen, welches Derivat der Formel (VII) man in Gegenwart einer Base mit einer wäßrigen Lösung eines Alkalimetallpersulfats umsetzt und dann mit konzentrierter Schwefelsäure behandelt zur Bildung der Verbindung der Formel (IX):

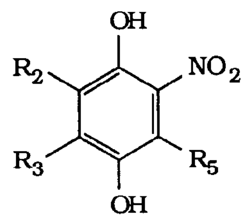

(IX)

in der:

$R_2$, $R_3$ und $R_5$ die bezüglich der Formel (I) angegebenen Bedeutungen besitzen, welche Derivate der Formel (IV), (VI), (VIII) und (IX) man einer katalytischen Hydrierung unterwirft,

zur Bildung der Verbindungen der Formel (X), (XI) und (XII), welche man unter einer inerten Atmosphäre aufbewahrt:

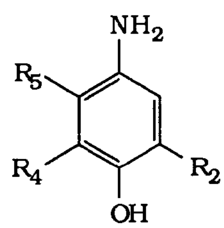

(X)

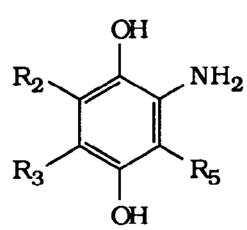

(XI)

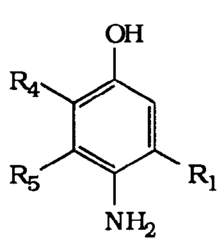

(XII)

worin

$R_1$, $R_2$, $R_3$, $R_4$ und $R_5$ die bezüglich der Formel (I) angegebenen Bedeutungen besitzen, welche Derivate der Formel (X), (XI) und (XII) man unter einer inerten Atmosphäre entweder mit einer Verbindung der Formel (XIII):

$$R—CO\text{-}O—CO\text{-}O—CH_2—CH_3 \quad (XIII)$$

in der R die bezüglich der Formel (I) angegebenen Bedeutungen besitzt, oder mit einer Verbindung der Formel (XIV) in Gegenwart einer organischen Base:

$$R—CO\text{-}Cl \quad (XIV)$$

in der R die bezüglich der Formel (I) angegebenen Bedeutungen besitzt, umsetzt, so daß man Verbindungen der Formeln (I/a), (I/b) bzw. (I/c) erhält, welche gemeinsam die Verbindungen der Formel (I) darstellen:

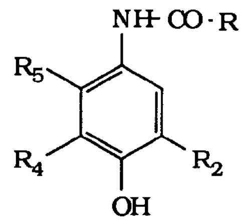

(I/a)

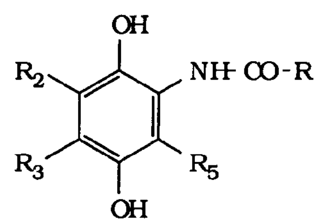

(I/b)

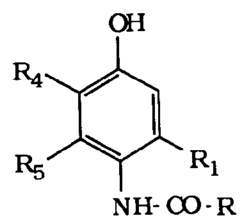

(I/c)

welche Verbindungen der Formeln (I/a), (I/b) und (I/ c) man mit Hilfe einer klassischen Reinigungsmethode reinigt und gegebenenfalls mit Hilfe klassischer Trennungsmethoden in die optischen Isomeren oder die cis/trans-Isomeren auftrennt und die man mit einer pharmazeutisch annehmbaren Base in ihre Additionssalze umwandeln kann.

2. Verfahren nach Anspruch 1 zur Herstellung von Verbindungen, worin $R_3$ eine Hydroxylgruppe darstellt, von denen Enantiomeren, Diastereoisomeren und Epimeren, von deren cis/trans-Isomeren und von deren Additionssalzen mit einer pharmazeutisch annehmbaren Base.

3. Verfahren nach Anspruch 1 zur Herstellung von Verbindungen, worin $R_1$ und $R_4$ gleichzeitig eine Hydroxylgruppe bedeuten, von deren Enantiomeren, Diastereoisomeren und Epimeren, von deren cis/trans-Isomeren sowie von deren Additionssalzen mit einer pharmazeutisch annehmbaren Base.

4. Verfahren nach Anspruch 1 zur Herstellung von Verbindungen, worin $R_3$ eine Hydroxylgruppe und $R_2$, $R_4$ und $R_5$ gleichzeitig eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

5. Verfahren nach Anspruch 1 zur Herstellung von Verbindungen, worin $R_3$ eine Hydroxylgruppe und $R_1$, $R_4$

und $R_5$ gleichzeitig eine Methylgruppe bedeuten, von deren Enantiomeren, Diastereoisomeren und Epimeren, von deren cis/trans-Isomeren und deren Additionssalzen mit einer pharmazeutisch annehmbaren Base.

6. Verfahren nach Anspruch 1 zur Herstellung von Verbindungen, worin $R_3$ eine Hydroxylgruppe, $R_2$ eine Methylgruppe und $R_4$ und $R_5$ gleichzeitig eine Methoxygruppe bedeuten, von deren Enantiomeren, Diastereoisomeren und Epimeren, von deren cis/trans-Isomeren sowie von deren Additionssalzen mit einer pharmazeutisch annehmbaren Base.

7. Verfahren nach Anspruch 1 zur Herstellung von Verbindungen, worin $R_1$ und $R_4$ gleichzeitig eine Hydroxylgruppe und $R_2$, $R_3$ und $R_5$ gleichzeitig eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

8. Verfahren nach Anspruch 1 zur Herstellung von Verbindungen, worin $R_1$ und $R_4$ gleichzeitig eine Hydroxylgruppe, $R_2$ und $R_3$ gleichzeitig eine Methoxygruppe und $R_5$ eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

9. Verfahren nach einem der Ansprüche 1, 2 oder 4 zur Herstellung der Verbindung 2,3,6-Trimethyl-4-(2-methylundecanamido)-phenol, von dessen Enantiomeren sowie von dessen Additionssalzen mit einer pharmazeutisch annehmbaren Base.

10. Verfahren nach einem der Ansprüche 1, 2 oder 4 zur Herstellung der Verbindung 2,3,6-Trimethyl-4-(2,2-dimethylundecanamido)-phenol sowie von dessen Additionssalzen mit einer pharmazeutisch annehmbaren Base.

11. Verfahren nach einem der Ansprüche 1, 2 oder 4 zur Herstellung der Verbindung 2,3,6-Trimethyl-4-(2-methyl-9-octadecenamido)-phenol, von dessen Enantiomeren, cis/trans-Isomeren sowie von dessen Additionssalzen mit einer pharmazeutisch annehmbaren Base.

12. Verfahren nach einem der Ansprüche 1, 2 oder 4 zur Herstellung der Verbindung 2,3,6-Trimethyl-4-(9,12-octadecadienamido)-phenol, von dessen cis/ trans-Isomeren sowie von dessen Additionssalzen mit einer pharmazeutisch annehmbaren Base.

13. Verfahren nach einem der Ansprüche 1, 2 oder 4 zur Herstellung der Verbindung 2,3,6-Trimethyl-4-(6,9,12-octadecatrienamido)-phenol, von dessen cis/trans-Isomeren sowie von dessen Additionssalzen mit einer pharmazeutisch annehmbaren Base.

**Patentansprüche für folgenden Vertragsstaat : GR**

1. Verfahren zur Herstellung der Verbindungen der Formel (I):

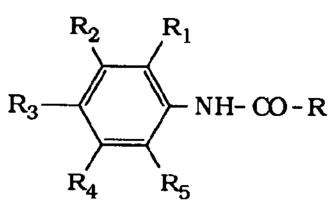

(I)

in der

R eine geradkettige oder verzweigte ($C_8C_{21}$)-Alkylgruppe, eine geradkettige oder verzweigte 1-[($C_8$-$C_{21}$)-Alkyl]-1-($C_3$-$C_6$)-Cycloalkylgruppe oder eine geradkettige oder verzweigte ($C_8$-$C_{21}$)-Alkenylgruppe, die eine bis drei Doppelbindungen aufweist,

und entweder

$R_3$ eine Hydroxylgruppe,

$R_1$ und $R_2$, die voneinander verschieden sind, Wasserstoffatome oder geradkettige oder verzweigte ($C_1$-$C_6$)-Alkylgruppen,

$R_4$ und $R_5$, die gleichartig oder verschieden sein können, eine geradkettige oder verzweigte ($C_1$-

$C_6$)-Alkylgruppe oder geradkettige oder verzweigte ($C_1$-$C_6$)-Alkoxygruppe,

oder

$R_1$ und $R_4$ gleichzeitig eine Hydroxylgruppe und

$R_2$, $R_3$ und $R_5$, die gleichartig oder verschieden sein können, geradkettige oder verzweigte ($C_1$-$C_6$)-Alkylgruppen oder geradkettige oder verzweigte ($C_1$-$C_6$)-Alkoxygruppen bedeuten,

von deren Enantiomeren, Diastereoisomeren und Epimeren, deren cis/trans-Isomeren sowie deren Additionssalzen mit einer pharmazeutisch annehmbaren Base,

**dadurch gekennzeichnet,** daß man als Ausgangsmaterial eine Verbindung der Formel (II)

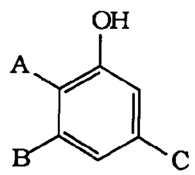

(II)

verwendet, in der die Substituenten A, B oder C, die gleichartig oder verschieden sein können, eine geradkettige oder verzweigte ($C_1$-$C_6$)-Alkylgruppe oder geradkettige oder verzweigte ($C_1$-$C_6$)-Alkoxygruppe bedeuten,

welche man

1

entweder

in Gegenwart einer Base mit einem Alkalimetallpersulfat zu einem Diphenol der Formel (II/a) umsetzt:

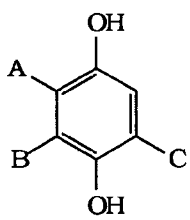

(II/a)

in der die Substituenten A, B und C die oben angegebenen Bedeutungen besitzen,

welches man mit Salpetersäure in essigsaurem Medium behandelt zur Bildung eines p-Benzochinons der Formel (III):

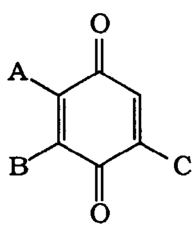

(III)

in der die Substituenten A, B und C die oben angegebenen Bedeutungen besitzen,

welches anschließend

entweder

wenn die Verbindungen der Formel (I), die man herzustellen wünscht, eine Gruppe $R_3$=OH aufweisen und in diesem Fall A, B und C durch $R_5$, $R_4$ bzw. $R_2$ ersetzt sein können,

in chlorwasserstoffsaurem Medium mit Hydroxylamin behandelt wird zur Bildung einer Verbindung der Formel (IV):

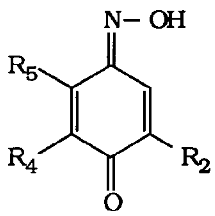

(IV)

in der

$R_2$, $R_4$ und $R_5$ die bezüglich der Formel (I) angegebenen Bedeutungen besitzen,

oder

wenn die Verbindungen der Formel (I), die man herzustellen wünscht, die Gruppen $R_1$=$R_4$=OH aufweisen und in diesem Fall A, B und C durch $R_2$, $R_3$ bzw. $R_5$ ersetzt sein können,

in wäßrigem Medium und in Gegenwart von Chlorwasserstoffsäure mit Salpetersäure behandelt zur Bildung der Verbindung der Formel (V):

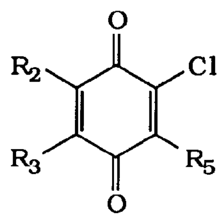

(V)

in der

$R_2$, $R_3$ und $R_5$ die bezüglich der Formel (I) angegebenen Bedeutungen besitzen,

welche man anschließend mit Natriumazid umsetzt zur Bildung der Verbindung der Formel (VI):

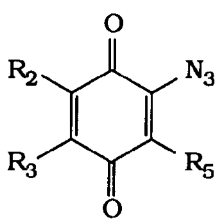

(VI)

in der $R_2$, $R_3$ und $R_5$ die bezüglich der Formel (I) angegebenen Bedeutungen besitzen,

**2**

oder

in chlorwasserstoffsaurem Medium und unter Verwendung von Lanthantrinitrat-hexahydrat als Katalysator mit Natriumnitrat umsetzt

zur Bildung einer Mischung der Verbindungen (VII) und (VIII), die man mit Hilfe einer klassischen Trennmethode trennt, für den Fall, wo die Verbindungen der Formel (I), die man herzustellen wünscht,

a die Gruppen $R_1$=$R_4$=OH besitzen und in diesem Fall A, B und C durch $R_2$, $R_3$ bzw. $R_5$ ersetzt sein können, oder

b eine Gruppe $R_3$=OH und in diesem Fall A, B und C durch $R_4$, $R_5$ bzw. $R_1$ ersetzt sein können,

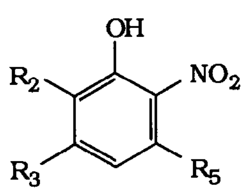

(VII)

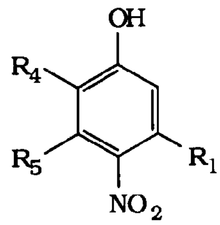

(VIII)

. worin:

. $R_1$, $R_2$, $R_3$, $R_4$ und $R_5$ die bezüglich der Formel (I) angegebenen Bedeutungen besitzen,

welches Derivat der Formel (VII) man in Gegenwart einer Base mit einer wäßrigen Lösung eines Alkalimetallpersulfats umsetzt und dann mit konzentrierter Schwefelsäure behandelt zur Bildung der Verbindung der Formel (IX):

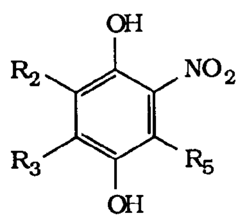

(IX)

in der:

$R_2$, $R_3$ und $R_5$ die bezüglich der Formel (I) angegebenen Bedeutungen besitzen, welche Derivate der Formel (IV), (VI), (VIII) und (IX) man einer katalytischen Hydrierung unterwirft,

zur Bildung der Verbindungen der Formel (X), (XI) und (XII), welche man unter einer inerten Atmosphäre aufbewahrt:

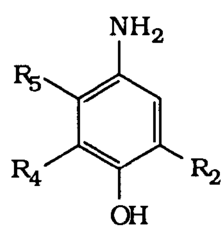

(X)

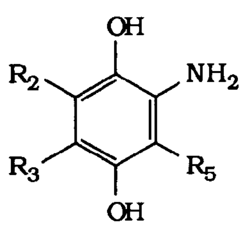

(XI)

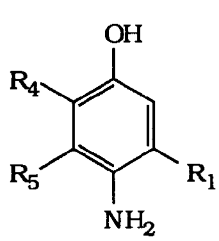

(XII)

worin
$R_1$, $R_2$, $R_3$, $R_4$ und $R_5$ die bezüglich der Formel (I) angegebenen Bedeutungen besitzen,
welche Derivate der Formel (X), (XI) und (XII) man unter einer inerten Atmosphäre entweder mit einer Verbindung der Formel (XIII):

$$R—CO\text{-}O—CO\text{-}O—CH_2—CH_3 \qquad (XIII)$$

in der R die bezüglich der Formel (I) angegebenen Bedeutungen besitzt,
oder mit einer Verbindung der Formel (XIV) in Gegenwart einer organischen Base:

$$R—CO\text{-}Cl \qquad (XIV)$$

in der R die bezüglich der Formel (I) angegebenen Bedeutungen besitzt, umsetzt, so daß man Verbindungen der Formeln (I/a), (I/b) bzw. (I/c) erhält, welche gemeinsam die Verbindungen der Formel (I) darstellen:

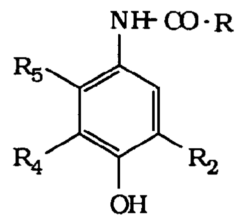

(I/a)

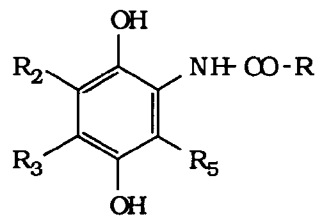

(I/b)

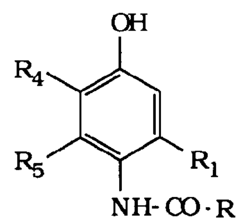

(I/c)

welche Verbindungen der Formeln (I/a), (I/b) und (I/c) man mit Hilfe einer klassischen Reinigungsmethode reinigt und gegebenenfalls mit Hilfe klassischer Trennungsmethoden in die optischen Isomeren oder die cis/trans-Isomeren auftrennt und die man mit einer pharmazeutisch annehmbaren Base in ihre Additionssalze umwandeln kann.

2. Verfahren nach Anspruch 1 zur Herstellung von Verbindungen, worin $R_3$ eine Hydroxylgruppe darstellt, von denen Enantiomeren, Diastereoisomeren und Epimeren, von deren cis/trans-Isomeren und von deren Additionssalzen mit einer pharmazeutisch annehmbaren Base.

3. Verfahren nach Anspruch 1 zur Herstellung von Verbindungen, worin $R_1$ und $R_4$ gleichzeitig eine Hydroxylgruppe bedeuten, von deren Enantiomeren, Diastereoisomeren und Epimeren, von deren cis/trans-Isomeren sowie von deren Additionssalzen mit einer pharmazeutisch annehmbaren Base.

4. Verfahren nach Anspruch 1 zur Herstellung von Verbindungen, worin $R_3$ eine Hydroxylgruppe und $R_2$, $R_4$ und $R_5$ gleichzeitig eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

5. Verfahren nach Anspruch 1 zur Herstellung von Verbindungen, worin $R_3$ eine Hydroxylgruppe und $R_1$, $R_4$

und $R_5$ gleichzeitig eine Methylgruppe bedeuten, von deren Enantiomeren, Diastereoisomeren und Epimeren, von deren cis/trans-Isomeren und deren Additionssalzen mit einer pharmazeutisch annehmbaren Base.

6. Verfahren nach Anspruch 1 zur Herstellung von Verbindungen, worin $R_3$ eine Hydroxylgruppe, $R_2$ eine Methylgruppe und $R_4$ und $R_5$ gleichzeitig eine Methoxygruppe bedeuten, von deren Enantiomeren, Diastereoisomeren und Epimeren, von deren cis/trans-Isomeren sowie von deren Additionssalzen mit einer pharmazeutisch annehmbaren Base.

7. Verfahren nach Anspruch 1 zur Herstellung von Verbindungen, worin $R_1$ und $R_4$ gleichzeitig eine Hydroxylgruppe und $R_2$, $R_3$ und $R_5$ gleichzeitig eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

8. Verfahren nach Anspruch 1 zur Herstellung von Verbindungen, worin $R_1$ und $R_4$ gleichzeitig eine Hydroxylgruppe, $R_2$ und $R_3$ gleichzeitig eine Methoxygruppe und $R_5$ eine Methylgruppe bedeuten, von deren Enantiomeren, Diastereoisomeren und Epimeren, von deren cis/trans-Isomeren sowie von deren Additionssalzen mit einer pharmazeutisch annehmbaren Base.

9. Verfahren nach einem der Ansprüche 1, 2 oder 4 zur Herstellung der Verbindung 2,3,6-Trimethyl-4-(2-methylundecanamido)-phenol, von dessen Enantiomeren sowie von dessen Additionssalzen mit einer pharmazeutisch annehmbaren Base.

10. Verfahren nach einem der Ansprüche 1, 2 oder 4 zur Herstellung der Verbindung 2,3,6-Trimethyl-4-(2,2-dimethylundecanamido)-phenol sowie von dessen Additionssalzen mit einer pharmazeutisch annehmbaren Base.

11. Verfahren nach einem der Ansprüche 1, 2 oder 4 zur Herstellung der Verbindung 2,3,6-Trimethyl-4-(2-methyl-9-octadecenamido)-phenol, von dessen Enantiomeren, cis/trans-Isomeren sowie von dessen Additionssalzen mit einer pharmazeutisch annehmbaren Base.

12. Verfahren nach einem der Ansprüche 1, 2 oder 4 zur Herstellung der Verbindung 2,3,6-Trimethyl-4-(9,12-octadecadienamido)-phenol, von dessen cis/trans-Isomeren sowie von dessen Additionssalzen mit einer pharmazeutisch annehmbaren Base.

13. Verfahren nach einem der Ansprüche 1, 2 oder 4 zur Herstellung der Verbindung 2,3,6-Trimethyl-4-(6,9,12-octadecatrienamido)-phenol, von dessen cis/trans-Isomeren sowie von dessen Additionssalzen mit einer pharmazeutisch annehmbaren Base.

## Claims

### Claims for the following Contracting States : AT, BE, CH, DE, DK, FR, GB, IT, LI, LU, NL, PT, SE

1. Compounds of formula (I):

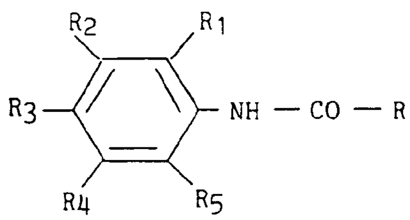

(I)

wherein :

R represents a straight-chain or branched ($C_8$-$C_{21}$)-alkyl group, a straight-chain or branched 1-[($C_8$-$C_{21}$)alkyl-($C_3$-$C_6$)cycloalk-1-yl group, or a straight-chain or branched ($C_8$-$C_{21}$)alkenyl group con-

taining from one to three double bonds,

and <u>either</u>

| | |
|---|---|
| $R_3$ | represents a hydroxy group, |
| $R_1$ and $R_2$, | which are different, represent a hydrogen atom or a straight-chain or branched ($C_1$-$C_6$)alkyl group, and |
| $R_4$ and $R_5$, | which are the same or different, represent a straight-chain or branched ($C_1$-$C_6$)alkyl group or a straight-chain or branched ($C_1$-$C_6$)alkoxy group, |

<u>or</u>

| | |
|---|---|
| $R_1$ and $R_4$ | each represents a hydroxy group, and |
| $R_2$, $R_3$ and $R_5$, | which are the same or different, represent a straight-chain or branched ($C_1$-$C_6$)alkyl group or a straight-chain or branched ($C_1$-$C_6$)alkoxy group, their enantiomers, diastereoisomers and epimers, their cis/trans isomers, and also their addition salts with a pharmaceutically acceptable base. |

**2.** Compounds according to claim 1 wherein $R_3$ represents a hydroxy group, their enantiomers, diastereoisomers and epimers, their cis/trans isomers, and also their addition salts with a pharmaceutically acceptable base.

**3.** Compounds according to claim 1 wherein $R_1$ and $R_4$ each represents a hydroxy group, their enantiomers, diastereoisomers and epimers, their cis/trans isomers, and also their addition salts with a pharmaceutically acceptable base.

**4.** Compounds according to claim 1 wherein $R_3$ represents a hydroxy group, and $R_2$, $R_4$ and $R_5$ each represents a methyl group, their enantiomers, diastereoisomers and epimers, their cis/trans isomers, and also their addition salts with a pharmaceutically acceptable base.

**5.** Compounds according to claim 1 wherein $R_3$ represents a hydroxy group, and $R_1$, $R_4$ and $R_5$ each represents a methyl group, their enantiomers, diastereoisomers and epimers, their cis/trans isomers, and also their addition salts with a pharmaceutically acceptable base.

**6.** Compounds according to claim 1 wherein $R_3$ represents a hydroxy group, $R_2$ represents a methyl group, and $R_4$ and $R_5$ each represents a methoxy group, their enantiomers, diastereoisomers and epimers, their cis/trans isomers, and also their addition salts with a pharmaceutically acceptable base.

**7.** Compounds according to claim 1 wherein $R_1$ and $R_4$ each represents a hydroxy group, and $R_2$, $R_3$ and $R_5$ each represents a methyl group, their enantiomers, diastereoisomers and epimers, their cis/trans isomers, and also their addition salts with a pharmaceutically acceptable base.

**8.** Compounds according to claim 1 wherein $R_1$ and $R_4$ each represents a hydroxy group, $R_2$ and $R_3$ each represents a methoxy group, and $R_5$ represents a methyl group, their enantiomers, diastereoisomers and epimers, their cis/trans isomers, and also their addition salts with a pharmaceutically acceptable base.

**9.** Compound according to any one of claims 1, 2 and 4, which is 2,3,6-trimethyl-4-(2-methylundecanamido)phenol, its enantiomers and also its addition salts with a pharmaceutically acceptable base.

**10.** Compound according to any one of claims 1, 2 and 4, which is 2,3,6-trimethyl-4-(2,2-dimethylundecanamido)phenol, and also its addition salts with a pharmaceutically acceptable base.

**11.** Compound according to any one of claims 1, 2 and 4, which is 2,3,6-trimethyl-4-(2-methyl-9-octadecenamido)phenol, its enantiomers, cis/trans isomers and also its addition salts with a pharmaceutically acceptable base.

**12.** Compound according to any one of claims 1, 2 and 4, which is 2,3,6-trimethyl-4-(9,12-octadecadienamido)phenol, its cis/trans isomers and also its addition salts with a pharmaceutically acceptable base.

**13.** Compound according to any one of claims 1, 2 and 4, which is 2,3,6-trimethyl-4-(6,9,12-octadecatrienamido)phenol, its cis/trans isomers and also its addition salts with a pharmaceutically acceptable base.

**14.** Process for the preparation of compounds of formula (I), characterised in that there is used as starting material a compound of formula (II)

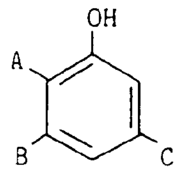

(II)

wherein the substituents A, B and C, which are the same or different, each represents a straight-chain or branched ($C_1$-$C_6$)alkyl or straight-chain or branched ($C_1$-$C_6$)alkoxy group,
which is reacted :
1
either
in the presence of an alkali metal persulphate in the presence of a base, to yield the diphenol of formula (II/a) :

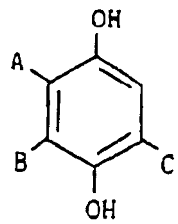

(II/a)

wherein the substituents A, B and C are as defined hereinbefore,
which is treated with nitric acid in acetic acid medium to yield a p-benzoquinone of formula (III) :

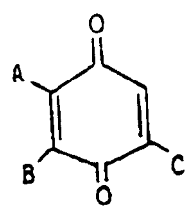

(III)

wherein the substituents A, B and C are as defined hereinbefore,
which is then treated
either
in the case where it is desired to obtain compounds of formula (I) that possess a $R_3$=OH group, in which case A, B and C may be replaced by $R_5$, $R_4$ and $R_2$ respectively,
in the presence of hydroxylamine in hydrochloric acid medium to yield the compound of formula IV :

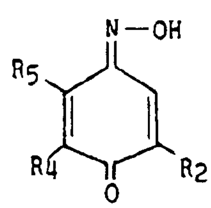

(IV)

wherein :
$R_2$, $R_4$ and $R_5$ have the same meanings as in formula (I)
or
in the case where it is desired to obtain compounds of formula (I) that possess $R_1$=$R_4$=OH groups, in which case A, B and C may be replaced by $R_2$, $R_3$ and $R_5$ respectively,
in the presence of hydrochloric acid in aqueous medium then in the presence of nitric acid to yield

the compound of formula (V) :

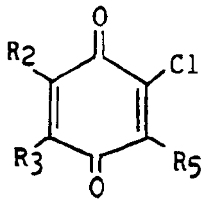

(V)

wherein :
$R_2$, $R_3$ and $R_5$ have the same meanings as in formula (I),
which is then reacted in the presence of sodium azide,
to yield the compound of formula (VI) :

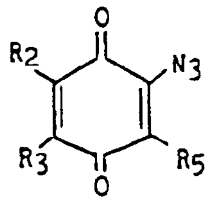

(VI)

wherein $R_2$, $R_3$ and $R_5$ have the same meanings as in formula (I)

2
or

in the presence of sodium nitrate and using lanthanum trinitrate hexahydrate as catalyst in hydrochloric acid medium,

to yield a mixture of compounds (VII) and (VIII), which are separated by a conventional method of separation,

in the case where it is desired to obtain compounds of formula (I) that possess :

a $R_1=R_4=OH$ groups, in which case A, B and C may be replaced by $R_2$, $R_3$ and $R_5$ respectively,
or
b a $R_3=OH$ group, in which case A, B and C may be replaced by $R_4$, $R_5$ and $R_1$ respectively

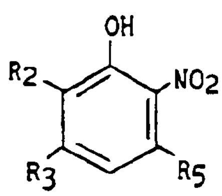

(VII)

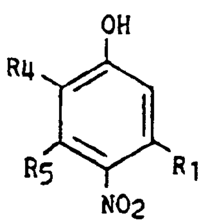

(VIII)

. wherein :
. $R_1$, $R_2$, $R_3$, $R_4$ and $R_5$ have the same meanings as in formula (I),

which compound of formula (VII) is reacted with an aqueous alkali metal persulphate solution in the presence of a base and then treated with concentrated sulphuric acid,
to yield the compound of formula (IX) :

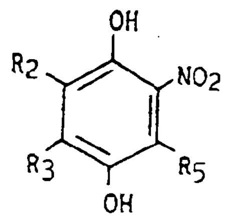

(IX)

wherein :
$R_2$, $R_3$ and $R_5$ have the same meanings as in formula (I),
which compounds of formulae (IV), (VI), (VIII) and (IX) are subjected to catalytic hydrogenation to yield the compounds of formulae (X), (XI) and (XII), which are kept under an inert atmosphere :

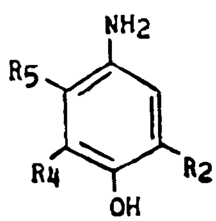

(X)

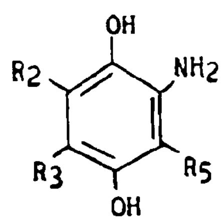

(XI)

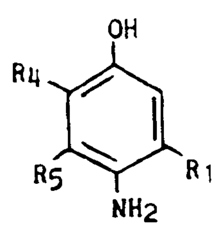

(XII)

wherein :
$R_1$, $R_2$, $R_3$, $R_4$ and $R_5$ have the same meanings as in formula (I),
which compounds of formula (X), (XI) and (XII) are reacted under an inert atmosphere :
either with a compound of formula (XIII) :

$$R-CO-O-CO-O-CH_2-CH_3 \qquad (XIII)$$

wherein R has the same meaning as in formula (I),
or with a compound of formula (XIV), in the presence of an organic base :

$$R-CO-Cl \qquad (XIV)$$

wherein R has the same meaning as in formula (I),
to yield the compounds (I/a), (I/b) and (I/c) respectively, which constitute the totality of the compounds of formula (I):

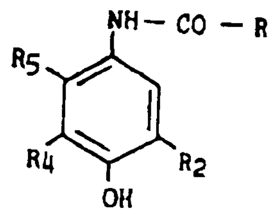

(I/a)

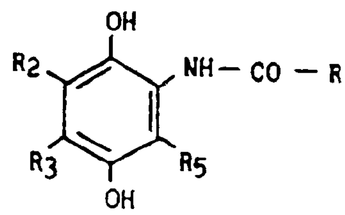

(I/b)

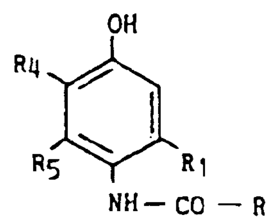

(I/c)

which compounds of formulae (I/a), (I/b) and (I/c) are purified by a conventional method of purification and of which, where applicable, the optical or cis/trans isomers are separated by conventional methods of separation, and which may be converted into their addition salts with a pharmaceutically acceptable base.

**15.** Pharmaceutical compositions comprising as active ingredient a compound according to any one of claims 1 to 13, alone or in combination with one or more pharmaceutically acceptable, inert non-toxic carriers.

**16.** Pharmaceutical compositions according to claim 15 which are useful in the treatment of dyslipidaemiae and arteriosclerosis.

**Claims for the following Contracting State : ES**

**1.** Process for the preparation of compounds of formula (I) :

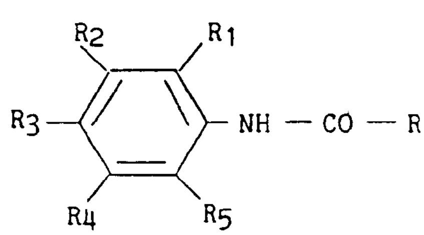

(I)

wherein :

R represents a straight-chain or branched ($C_8$-$C_{21}$)-alkyl group, a straight-chain or branched 1-[($C_8$-$C_{21}$)alkyl-($C_3$-$C_6$)cycloalk-1-yl group, or a straight-chain or branched ($C_8$-$C_{21}$)alkenyl group containing from one to three double bonds,

and <u>either</u>

$R_3$ represents a hydroxy group,

$R_1$ and $R_2$, which are different, represent a hydrogen atom or a straight-chain or branched ($C_1$-

$C_6$)alkyl group, and

$R_4$ and $R_5$, which are the same or different, represent a straight-chain or branched ($C_1$-$C_6$)alkyl group or a straight-chain or branched ($C_1$-$C_6$)alkoxy group,

or

$R_1$ and $R_4$ each represents a hydroxy group, and

$R_2$, $R_3$ and $R_5$, which are the same or different, represent a straight-chain or branched ($C_1$-$C_6$)alkyl group or a straight-chain or branched ($C_1$-$C_6$)alkoxy group,

their enantiomers, diastereoisomers and epimers, their cis/trans isomers, and also their addition salts with a pharmaceutically acceptable base,

characterised in that there is used as starting material a compound of formula (II)

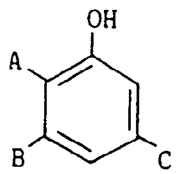

(II)

wherein the substituents A, B and C, which are the same or different, each represents a straight-chain or branched ($C_1$-$C_6$)alkyl or straight-chain or branched ($C_1$-$C_6$)alkoxy group,

which is reacted :

1

either

in the presence of an alkali metal persulphate in the presence of a base, to yield the diphenol of formula (II/a) :

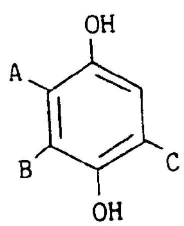

(II/a)

wherein the substituents A, B and C are as defined hereinbefore,

which is treated with nitric acid in acetic acid medium to yield a p-benzoquinone of formula (III) :

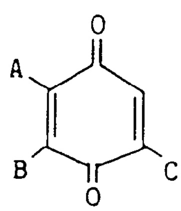

(III)

wherein the substituents A, B and C are as defined hereinbefore,

which is then treated

either

in the case where it is desired to obtain compounds of formula (I) that possess a $R_3$=OH group, in which case A, B and C may be replaced by $R_5$, $R_4$ and $R_2$ respectively,

in the presence of hydroxylamine in hydrochloric acid medium to yield the compound of formula IV :

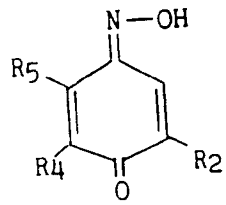

(IV)

wherein :
$R_2$, $R_4$ and $R_5$ have the same meanings as in formula (I)

or

in the case where it is desired to obtain compounds of formula (I) that possess $R_1=R_4=OH$ groups, in which case A, B and C may be replaced by $R_2$, $R_3$ and $R_5$ respectively,

in the presence of hydrochloric acid in aqueous medium then in the presence of nitric acid to yield the compound of formula (V) :

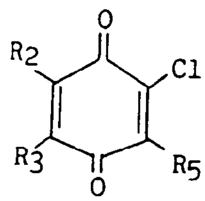

(V)

wherein :
$R_2$, $R_3$ and $R_5$ have the same meanings as in formula (I),
which is then reacted in the presence of sodium azide,
to yield the compound of formula (VI) :

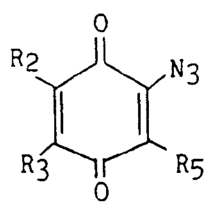

(VI)

wherein $R_2$, $R_3$ and $R_5$ have the same meanings as in formula (I)

2

or

in the presence of sodium nitrate and using lanthanum trinitrate hexahydrate as catalyst in hydrochloric acid medium,

to yield a mixture of compounds (VII) and (VIII), which are separated by a conventional method of separation,

in the case where it is desired to obtain compounds of formula (I) that possess :

a $R_1=R_4=OH$ groups, in which case A, B and C may be replaced by $R_2$, $R_3$ and $R_5$ respectively,

or

b a $R_3=OH$ group, in which case A, B and C may be replaced by $R_4$, $R_5$ and $R_1$ respectively

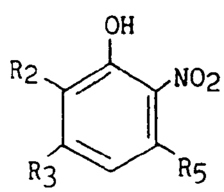

(VII)

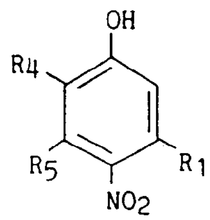

(VIII)

. wherein :

. $R_1$, $R_2$, $R_3$, $R_4$ and $R_5$ have the same meanings as in formula (I),

which compound of formula (VII) is reacted with an aqueous alkali metal persulphate solution in the presence of a base and then treated with concentrated sulphuric acid,
to yield the compound of formula (IX) :

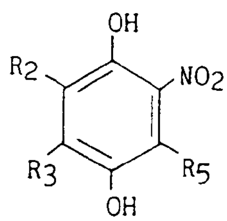

(IX)

wherein :
$R_2$, $R_3$ and $R_5$ have the same meanings as in formula (I),
which compounds of formulae (IV), (VI), (VIII) and (IX) are subjected to catalytic hydrogenation to yield the compounds of formulae (X), (XI) and (XII), which are kept under an inert atmosphere :

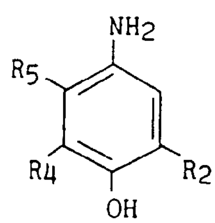

(X)

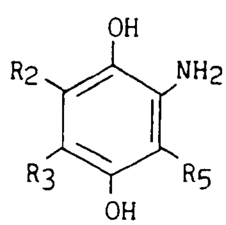

(XI)

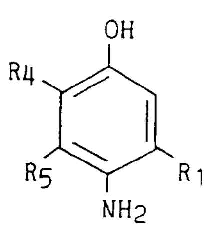

(XII)

wherein :
$R_1$, $R_2$, $R_3$, $R_4$ and $R_5$ have the same meanings as in formula (I),
which compounds of formula (X), (XI) and (XII) are reacted under an inert atmosphere :
either with a compound of formula (XIII) :

$$R - CO - O - CO - O - CH_2 - CH_3 \qquad (XIII)$$

wherein R has the same meaning as in formula (I),
or with a compound of formula (XIV), in the presence of an organic base :

$$R - CO - Cl \qquad (XIV)$$

wherein R has the same meaning as in formula (I),
to yield the compounds (I/a), (I/b) and (I/c) respectively, which constitute the totality of the compounds of formula (I):

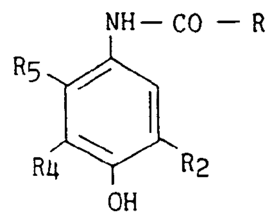

(I/a)

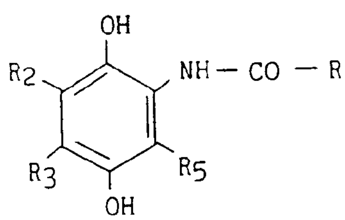

(I/b)

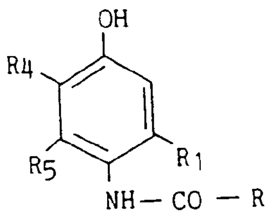

(I/c)

which compounds of formulae (I/a), (I/b) and (I/c) are purified by a conventional method of purification and of which, where applicable, the optical or cis/trans isomers are separated by conventional methods of separation, and which may be converted into their addition salts with a pharmaceutically acceptable base.

2. Process according to claim 1 for the preparation of compounds wherein $R_3$ represents a hydroxy group, their enantiomers, diastereoisomers and epimers, their cis/trans isomers, and also their addition salts with a pharmaceutically acceptable base.

3. Process according to claim 1 for the preparation of compounds wherein $R_1$ and $R_4$ each represents a hydroxy group, their enantiomers, diastereoisomers and epimers, their cis/trans isomers, and also their addition salts with a pharmaceutically acceptable base.

4. Process according to claim 1 for the preparation of compounds wherein $R_3$ represents a hydroxy group, and $R_2$, $R_4$ and $R_5$ each represents a methyl group, their enantiomers, diastereoisomers and epimers, their cis/trans isomers, and also their addition salts with a pharmaceutically acceptable base.

5. Process according to claim 1 for the preparation of compounds wherein $R_3$ represents a hydroxy group,

and $R_1$, $R_4$ and $R_5$ each represents a methyl group, their enantiomers, diastereoisomers and epimers, their cis/trans isomers, and also their addition salts with a pharmaceutically acceptable base.

6. Process according to claim 1 for the preparation of compounds wherein $R_3$ represents a hydroxy group, $R_2$ represents a methyl group, and $R_4$ and $R_5$ each represents a methoxy group, their enantiomers, diastereoisomers and epimers, their cis/trans isomers, and also their addition salts with a pharmaceutically acceptable base.

7. Process according to claim 1 for the preparation of compounds wherein $R_1$ and $R_4$ each represents a hydroxy group, and $R_2$, $R_3$ and $R_5$ each represents a methyl group, their enantiomers, diastereoisomers and epimers, their cis/trans isomers, and also their addition salts with a pharmaceutically acceptable base.

8. Process according to claim 1 for the preparation of compounds wherein $R_1$ and $R_4$ each represents a hydroxy group, $R_2$ and $R_3$ each represents a methoxy group, and $R_5$ represents a methyl group, their enantiomers, diastereoisomers and epimers, their cis/trans isomers, and also their addition salts with a pharmaceutically acceptable base.

9. Process according to any one of claims 1, 2 and 4 for the preparation of the compound 2,3,6-trimethyl-4-(2-methylundecanamido)phenol, its enantiomers and also its addition salts with a pharmaceutically acceptable base.

10. Process according to any one of claims 1, 2 and 4 for the preparation of the compound 2,3,6-trimethyl-4-(2,2-dimethylundecanamido)phenol, and also its addition salts with a pharmaceutically acceptable base.

11. Process according to any one of claims 1, 2 and 4 for the preparation of the compound 2,3,6-trimethyl-4-(2-methyl-9-octadecenamido)phenol, its enantiomers, cis/trans isomers and also its addition salts with a pharmaceutically acceptable base.

12. Process according to any one of claims 1, 2 and 4 for the preparation of the compound 2,3,6-trimethyl-4-(9,12-octadecadienamido)phenol, its cis/trans isomers and also its addition salts with a pharmaceutically acceptable base.

13. Process according to any one of claims 1, 2 and 4 for the preparation of the compound 2,3,6-trimethyl-4-(6,9,12-octadecatrienamido)phenol, its cis/trans isomers and also its addition salts with a pharmaceutically acceptable base.

**Claims for the following Contracting State : GR**

1. Process for the preparation of compounds of formula (I) :

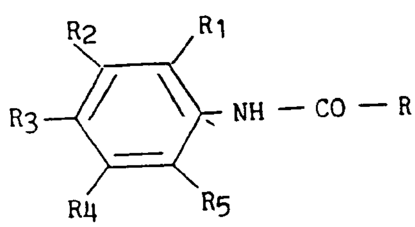

(I)

wherein :

R represents a straight-chain or branched ($C_8$-$C_{21}$)-alkyl group, a straight-chain or branched 1-[($C_8$-$C_{21}$)alkyl-($C_3$-$C_6$)cycloalk-l-yl group, or a straight-chain or branched ($C_8$-$C_{21}$)alkenyl group containing from one to three double bonds,

and <u>either</u>

$R_3$ represents a hydroxy group,

$R_1$ and $R_2$, which are different, represent a hydrogen atom or a straight-chain or branched ($C_1$-$C_6$)alkyl group, and

$R_4$ and $R_5$, which are the same or different, represent a straight-chain or branched ($C_1$-$C_6$)alkyl

group or a straight-chain or branched ($C_1$-$C_6$)alkoxy group,

or

$R_1$ and $R_4$ each represents a hydroxy group, and

$R_2$, $R_3$ and $R_5$, which are the same or different, represent a straight-chain or branched ($C_1$-$C_6$)alkyl group or a straight-chain or branched ($C_1$-$C_6$)alkoxy group,

their enantiomers, diastereoisomers and epimers, their cis/trans isomers, and also their addition salts with a pharmaceutically acceptable base,

characterised in that there is used as starting material a compound of formula (II)

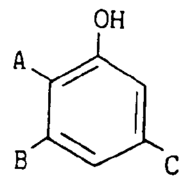

(II)

wherein the substituents A, B and C, which are the same or different, each represents a straight-chain or branched ($C_1$-$C_6$)alkyl or straight-chain or branched ($C_1$-$C_6$)alkoxy group,

which is reacted :

1

either

in the presence of an alkali metal persulphate in the presence of a base, to yield the diphenol of formula (II/a) :

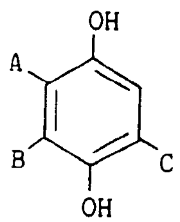

(II/a)

wherein the substituents A, B and C are as defined hereinbefore,

which is treated with nitric acid in acetic acid medium to yield a p-benzoquinone of formula (III) :

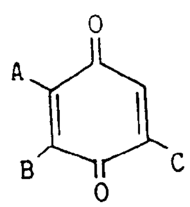

(III)

wherein the substituents A, B and C are as defined hereinbefore,

which is then treated

either

in the case where it is desired to obtain compounds of formula (I) that possess a $R_3$=OH group, in which case A, B and C may be replaced by $R_5$, $R_4$ and $R_2$ respectively,

in the presence of hydroxylamine in hydrochloric acid medium to yield the compound of formula IV :

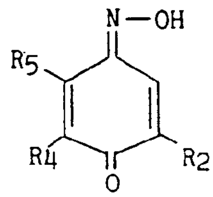

(IV)

wherein :

$R_2$, $R_4$ and $R_5$ have the same meanings as in formula (I)

or

in the case where it is desired to obtain compounds of formula (I) that possess $R_1=R_4=OH$ groups, in which case A, B and C may be replaced by $R_2$, $R_3$ and $R_5$ respectively,

in the presence of hydrochloric acid in aqueous medium then in the presence of nitric acid to yield the compound of formula (V) :

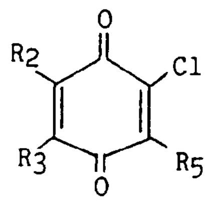

(V)

wherein :

$R_2$, $R_3$ and $R_5$ have the same meanings as in formula (I),
which is then reacted in the presence of sodium azide,
to yield the compound of formula (VI) :

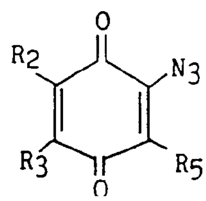

(VI)

wherein $R_2$, $R_3$ and $R_5$ have the same meanings as in formula (I)

2

or

in the presence of sodium nitrate and using lanthanum trinitrate hexahydrate as catalyst in hydrochloric acid medium,
to yield a mixture of compounds (VII) and (VIII), which are separated by a conventional method of separation,

in the case where it is desired to obtain compounds of formula (I) that possess :

a $R_1=R_4=OH$ groups, in which case A, B and C may be replaced by $R_2$, $R_3$ and $R_5$ respectively,

or

b a $R_3=OH$ group, in which case A, B and C may be replaced by $R_4$, $R_5$ and $R_1$ respectively

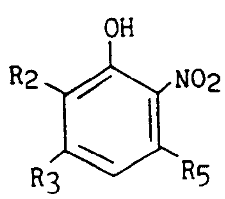

(VII)

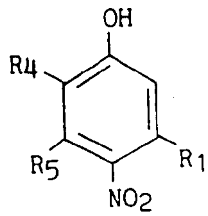

(VIII)

- wherein :
- $R_1$, $R_2$, $R_3$, $R_4$ and $R_5$ have the same meanings as in formula (I),

which compound of formula (VII) is reacted with an aqueous alkali metal persulphate solution in the presence of a base and then treated with concentrated sulphuric acid,
to yield the compound of formula (IX) :

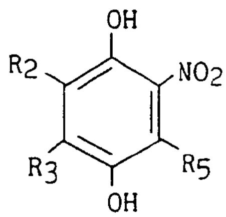

(IX)

wherein :
$R_2$, $R_3$ and $R_5$ have the same meanings as in formula (I),
which compounds of formulae (IV), (VI), (VIII) and (IX) are subjected to catalytic hydrogenation to yield the compounds of formulae (X), (XI) and (XII), which are kept under an inert atmosphere :

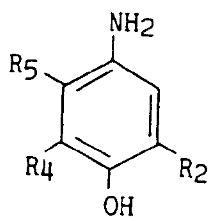

(X)

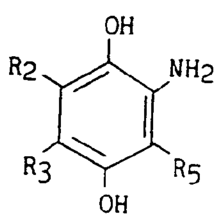

(XI)

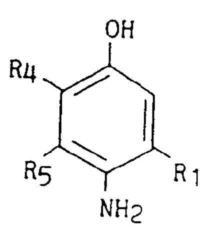

(XII)

wherein :
$R_1$, $R_2$, $R_3$, $R_4$ and $R_5$ have the same meanings as in formula (I),
which compounds of formula (X), (XI) and (XII) are reacted under an inert atmosphere :
either with a compound of formula (XIII) :

$$R - CO - O - CO - O - CH_2 - CH_3 \qquad (XIII)$$

wherein R has the same meaning as in formula (I),
or with a compound of formula (XIV), in the presence of an organic base :

$$R - CO - Cl \qquad (XIV)$$

wherein R has the same meaning as in formula (I),
to yield the compounds (I/a), (I/b) and (I/c) respectively, which constitute the totality of the compounds of formula (I):

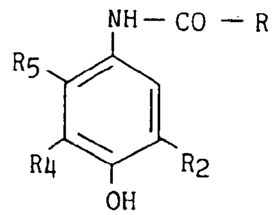

(I/a)

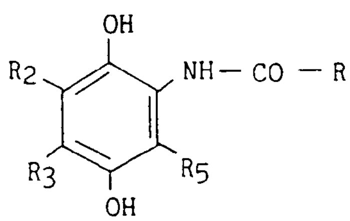

(I/b)

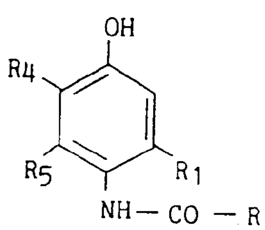

(I/c)

which compounds of formulae (I/a), (I/b) and (I/c) are purified by a conventional method of purification and of which, where applicable, the optical or cis/trans isomers are separated by conventional methods of separation, and which may be converted into their addition salts with a pharmaceutically acceptable base.

2. Process according to claim 1 for the preparation of compounds wherein $R_3$ represents a hydroxy group, their enantiomers, diastereoisomers and epimers, their cis/trans isomers, and also their addition salts with a pharmaceutically acceptable base.

3. Process according to claim 1 for the preparation of compounds wherein $R_1$ and $R_4$ each represents a hydroxy group, their enantiomers, diastereoisomers and epimers, their cis/trans isomers, and also their addition salts with a pharmaceutically acceptable base.

4. Process according to claim 1 for the preparation of compounds wherein $R_3$ represents a hydroxy group, and $R_2$, $R_4$ and $R_5$ each represents a methyl group, their enantiomers, diastereoisomers and epimers, their cis/trans isomers, and also their addition salts with a pharmaceutically acceptable base.

5. Process according to claim 1 for the preparation of compounds wherein $R_3$ represents a hydroxy group, and $R_1$, $R_4$ and $R_5$ each represents a methyl group, their enantiomers, diastereoisomers and epimers,

their cis/trans isomers, and also their addition salts with a pharmaceutically acceptable base.

6. Process according to claim 1 for the preparation of compounds wherein $R_3$ represents a hydroxy group, $R_2$ represents a methyl group, and $R_4$ and $R_5$ each represents a methoxy group, their enantiomers, diastereoisomers and epimers, their cis/trans isomers, and also their addition salts with a pharmaceutically acceptable base.

7. Process according to claim 1 for the preparation of compounds wherein $R_1$ and $R_4$ each represents a hydroxy group, and $R_2$, $R_3$ and $R_5$ each represents a methyl group, their enantiomers, diastereoisomers and epimers, their cis/trans isomers, and also their addition salts with a pharmaceutically acceptable base.

8. Process according to claim 1 for the preparation of compounds wherein $R_1$ and $R_4$ each represents a hydroxy group, $R_2$ and $R_3$ each represents a methoxy group, and $R_5$ represents a methyl group, their enantiomers, diastereoisomers and epimers, their cis/trans isomers, and also their addition salts with a pharmaceutically acceptable base.

9. Process according to any one of claims 1, 2 and 4 for the preparation of the compound 2,3,6-trimethyl-4-(2-methylundecanamido)phenol, its enantiomers and also its addition salts with a pharmaceutically acceptable base.

10. Process according to any one of claims 1, 2 and 4 for the preparation of the compound 2,3,6-trimethyl-4-(2,2-dimethylundecanamido)phenol, and also its addition salts with a pharmaceutically acceptable base.

11. Process according to any one of claims 1, 2 and 4 for the preparation of the compound 2,3,6-trimethyl-4-(2-methyl-9-octadecenamido)phenol, its enantiomers, cis/trans isomers and also its addition salts with a pharmaceutically acceptable base.

12. Process according to any one of claims 1, 2 and 4 for the preparation of the compound 2,3,6-trimethyl-4-(9,12-octadecadienamido)phenol, its cis/trans isomers and also its addition salts with a pharmaceutically acceptable base.

13. Process according to any one of claims 1, 2 and 4 for the preparation of the compound 2,3,6-trimethyl-4-(6,9,12-octadecatrienamido)phenol, its cis/trans isomers and also its addition salts with a pharmaceutically acceptable base.